# EUROPÄISCHE PATENTSCHRIFT

(11) **EP 2 906 661 B1**
(45) Veröffentlichungstag und Bekanntmachung des Hinweises auf die Patenterteilung: **26.10.2016**
(21) Anmeldenummer: 13762409.4
(22) Anmeldetag: 13.09.2013
(51) Int. Cl.: C09K 11/06, C07D 455/03, C07D 471/16, C07D 471/22, C07D 491/048, C07D 495/22, C07D 498/14, C09K 11/02, H01L 51/00, C07D 513/14, H01L 51/50, H01L 51/52

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX DESTINÉS À DES DISPOSITIFS ÉLECTROLUMINESCENTS ORGANIQUES

(30) Priorität: 11.10.2012 EP 12007040
(43) Veröffentlichungstag der Anmeldung: 19.08.2015
(73) Patentinhaber: Merck Patent GmbH, 64293 Darmstadt (DE)
(72) Erfinder: STOESSEL, Philipp, 60487 Frankfurt am Main (DE); PARHAM, Amir Hossain, 60486 Frankfurt am Main (DE); PFLUMM, Christof, 64291 Darmstadt-Arheilgen (DE); JATSCH, Anja, 60489 Frankfurt am Main (DE); EBERLE, Thomas, 76829 Landau (DE); KROEBER, Jonas Valentin, 60311 Frankfurt am Main (DE)
(86) Internationale Anmeldenummer: PCT/EP2013/002748
(87) Internationale Veröffentlichungsnummer: WO 2014/056567

(56) Entgegenhaltungen:
- EP-A1- 0 014 567
- EP-A1- 0 074 777
- DE-A1-102005 037 115

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Quanten- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Dies gilt insbesondere auch für OLEDs, welche im kürzerwelligen Bereich, beispielsweise grün, emittieren.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, etc. von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Lactame, z. B. gemäß WO 2011/116865, DE 102005037115A1 oder WO 2011/137951, als Matrixmaterialien für phosphoreszierende Emitter in organischen Elektrolumineszenzvorrichtungen eingesetzt. Generell sind hier weitere Verbesserungen wünschenswert, insbesondere in Bezug auf die Effizienz, die Lebensdauer und die thermische Stabilität der Materialien.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Elektronentransport- bzw. Lochblockiermaterial. Insbesondere ist es die Aufgabe der vorliegenden Erfindung, Matrixmaterialien bereitzustellen, welche sich für grün und rot und gegebenenfalls auch blau phosphoreszierende OLEDs eignen.

Überraschend wurde gefunden, dass die unten näher beschriebenen Verbindungen diese Aufgabe lösen und zu Verbesserungen der organischen Elektrolumineszenzvorrichtung führen, insbesondere hinsichtlich der Lebensdauer, der Effizienz und/oder der Betriebsspannung. Dies gilt insbesondere für rot und grün phosphoreszierende Elektrolumineszenzvorrichtungen, vor allem bei Einsatz der erfindungsgemäßen Verbindungen als Matrixmaterial. Die Materialien zeichnen sich weiterhin durch durch eine hohe Temperaturstabilität aus. Elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1), 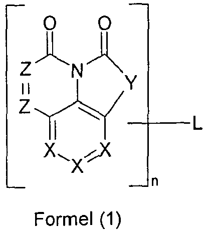 wobei für die verwendeten Symbole und Indizes gilt:
- X: ist bei jedem Auftreten gleich oder verschieden CR oder N; oder genau zwei benachbarte Gruppen X stehen zusammen für eine Gruppe, ausgewählt aus NR, O oder S, wodurch ein Fünfring entsteht;
- Y: ist bei jedem Auftreten gleich oder verschieden Z=Z, NR, wobei R ungleich H ist, O oder S;
- Z: ist bei jedem Auftreten gleich oder verschieden CR oder N oder die benachbarten Gruppen Z=Z stehen zusammen für eine Gruppe der Formel (2), 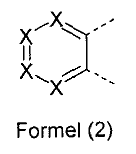 wobei X die oben genannten Bedeutungen aufweist und die gestrichelten Bindungen die Verknüpfung dieser Gruppe andeuten;
- L: ist für n = 1 nicht vorhanden und ist für n = 2 eine Einfachbindung oder eine bivalente Gruppe und für n = 3 eine trivalente Gruppe und für n = 4 eine tetravalente Gruppe und für n = 5 eine pentavalente Gruppe und für n = 6 eine hexavalente Gruppe; dabei ist L statt einer Gruppe R an einer beliebigen Stelle des Grundgerüsts gebunden;
- R: ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C=C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
- Ar¹: ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein;
- R¹: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine Alkylgruppe mit 1 bis 10 C-Atomen oder eine Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können;
- n: ist 1, 2, 3, 4, 5 oder 6;
wobei die folgende Verbindung von der Erfindung ausgenommen ist:

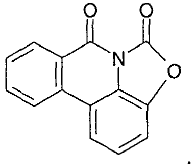

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cyclo-heptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenytthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Benzanthracen, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin; 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer bevorzugten Ausführungsform der Erfindung steht Y für eine Gruppe Z=Z. Bevorzugt ist also eine Verbindung der folgenden Formel (3), 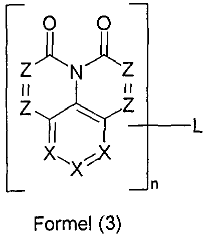 wobei die verwendeten Symbole und Indizes die oben genannten Bedeutungen aufweisen.

In einer weiteren bevorzugten Ausführungsform der Erfindung steht die Gruppe Z=Z für eine Gruppe der Formel (2), so dass eine Verbindung der folgenden Formel (4) entsteht, 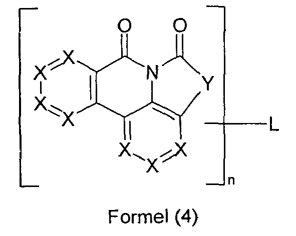 wobei die verwendeten Symbole und Indizes die oben genannten Bedeutungen aufweisen.

Eine bevorzugte Ausführungsform der Verbindungen der Formel (3) und (4) sind die Verbindungen der folgenden Formel (5), 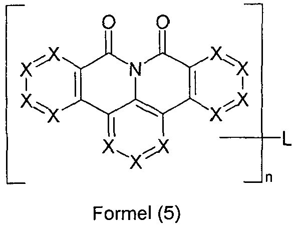 wobei die verwendeten Symbole und Indizes die oben genannten Bedeutungen aufweisen.

In einer weiteren bevorzugten Ausführungsform der Erfindung ist n = 1, 2 oder 3. Für n > 1 ist die Gruppe L bevorzugt in der para-Position zum Stickstoff gebunden. Es handelt sich also für n > 1 bevorzugt um eine Struktur der folgenden Formel (6), 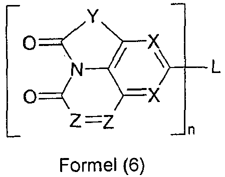 wobei n = 2, 3, 4, 5 oder 6 ist und die weiteren Symbole die oben genannten Bedeutungen aufweisen. Insbesondere haben die weiteren Symbole dieselben Bedeutungen, wie sie oben als bevorzugt aufgeführt sind.

In einer weiteren bevorzugten Ausführungsform der Erfindung steht maximal eine Gruppe X pro Cyclus für N und die weiteren Gruppen X stehen für CR. Besonders bevorzugt stehen alle Gruppen X für CR.

Bevorzugt sind somit die Verbindungen der folgenden Formel (7) und (8), 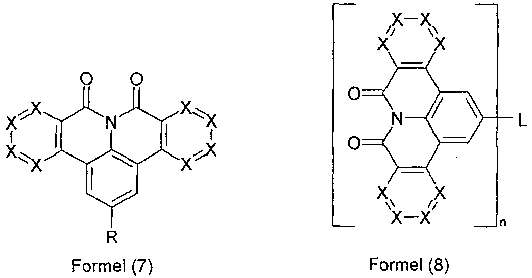 wobei n für 2 oder 3 steht, pro Cyclus maximal eine Gruppe X für N steht und die weiteren Gruppen X für CR stehen und die weiteren verwendeten Symbole die oben genannten Bedeutungen aufweisen.

Bevorzugte Ausführungsformen der Formeln (7) und (8) sind die Verbindungen der folgenden Formeln (7a) und (8a), 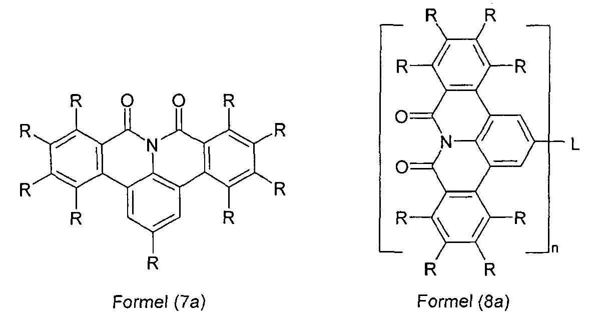 wobei die verwendeten Symbole und Indizes die oben genannten Bedeutungen aufweisen.

Bevorzugte Ausführungsformen der Formeln (7a) und (8a) sind die Verbindungen der folgenden Formeln (7b) und (8b), 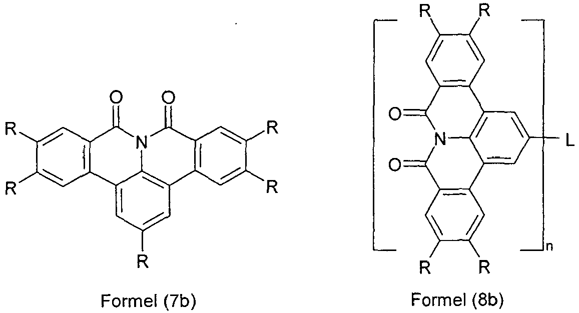 wobei die verwendeten Symbole und Indizes die oben genannten Bedeutungen aufweisen.

Besonders bevorzugte Ausführungsformen sind die Verbindungen der folgenden Formeln (7c) und (8c), 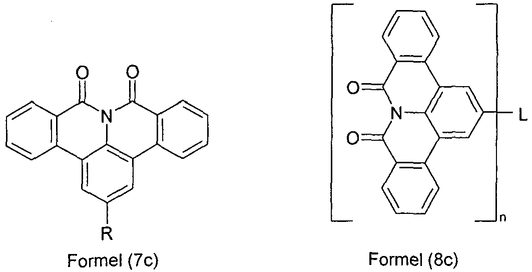 wobei die verwendeten Symbole und Indizes die oben genannten Bedeutungen aufweisen.

In einer weiteren bevorzugten Ausführungsform der Erfindung steht Y für eine Gruppe NR. Eine weitere bevorzugte Ausführungsform der Verbindungen der Formel (4) sind daher die Verbindungen der folgenden Formel (9), 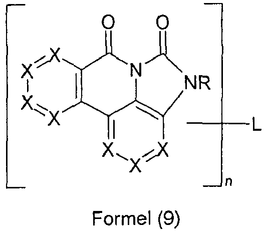 wobei die verwendeten Symbole und Indizes die oben genannten Bedeutungen aufweisen und der am Stickstoff gebundene Rest R ungleich Wasserstoff ist.

Bevorzugte Ausführungsformen der Verbindungen der Formel (9) sind die Verbindungen der folgenden Formeln (10) und (11), 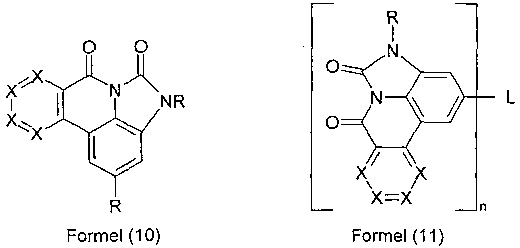 wobei n für 2 oder 3 steht, maximal eine Gruppe X für N steht und die weiteren Gruppen X für CR stehen, die weiteren verwendeten Symbole die oben genannten Bedeutungen aufweisen und der am Stickstoff gebundene Rest R ungleich Wasserstoff ist.

Bevorzugte Ausführungsformen der Formeln (10) und (11) sind die Verbindungen der folgenden Formeln (10a) und (11 a), 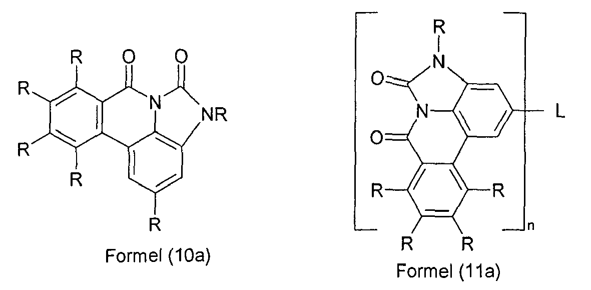 wobei die verwendeten Symbole und Indizes die oben genannten Bedeutungen aufweisen.

Bevorzugte Ausführungsformen der Formeln (10a) und (11a) sind die Verbindungen der folgenden Formeln (10b) und (11b), 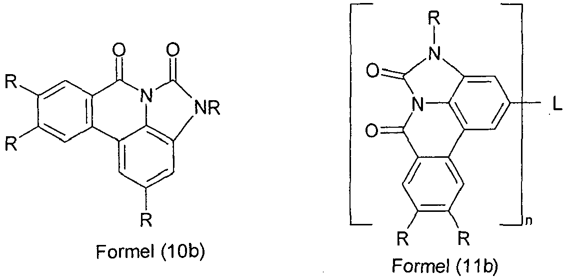 wobei die verwendeten Symbole und Indizes die oben genannten Bedeutungen aufweisen.

Besonders bevorzugte Ausführungsformen sind die Verbindungen der folgenden Formeln (10c) und (11 c), 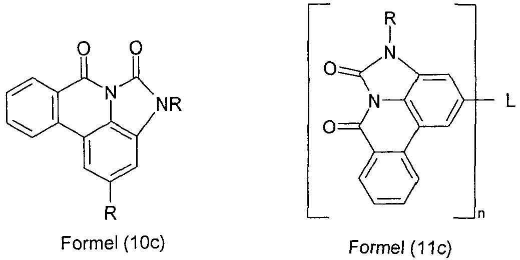 wobei die verwendeten Symbole und Indizes die oben genannten Bedeutungen aufweisen.

Bevorzugt sind weiterhin Strukturen, in denen der Rest R, der in Formel (9) bis (11) und (10a) bis (11 c) an den Stickstoff gebunden ist, für eine Gruppe C(=O)Ar¹ steht, die mit einem benachbarten Rest R einen Ring bildet. Dadurch sind dann Verbindungen der folgenden Formeln (12) zugänglich, 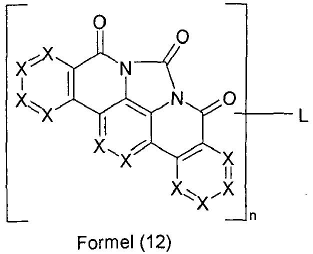 wobei die verwendeten Symbole und Indizes dieselben Bedeutungen aufweisen, wie oben beschrieben. Auch gelten für die in Formel (12) verwendeten Symbole und Indizes dieselben Bevorzugungen, wie oben beschrieben.

Bevorzugt steht L in den Verbindungen der Formel (1) bzw. den oben aufgeführten bevorzugten Verbindungen für n = 2 für eine Einfachbindung, CR₂, O, NR oder C(=O) oder für n = 3 für N oder für n ≥ 2 für für ein aromatisches oder heteroaromatisches Ringsystem mit 5 bis 24 aromatischen Ringatomen, das mit einem oder mehreren Resten R substituiert sein kann. Geeignete aromatische oder heteroaromatische Ringsysteme sind ortho-, meta- oder para-Phenylen, Fluoren, Spirobifluoren, Pyridin, Pyrimidin, Triazin, Carbazol, Dibenzofuran, Triphenylamin oder Kombinationen aus zwei oder drei dieser Gruppen. Dabei ist für n = 2 das aromatische bzw. heteroaromatische Ringsystem ein bivalentes System, für n = 3 ein trivalentes System, etc.. Besonders bevorzugt ist n = 2, und L steht für eine Einfachbindung.

Bevorzugt weisen die erfindungsgemäßen Verbindungen mindestens eine Gruppe R ungleich Wasserstoff oder Deuterium auf, und/oder es handelt sich um eine Verbindung mit n >1.

Der Rest R ungleich Wasserstoff ist bevorzugt ausgewählt aus der Gruppe bestehend aus einem aromatischen oder heteroaromatischen Ringsystem mit 5 bis 24 aromatischen Ringatomen, welches durch einen oder mehrere Reste R¹ substituiert sein kann. Dies gilt insbesondere auch für den Rest R, der an ein Stickstoffatom gebunden ist, wenn Y für NR steht. Geeignete Aryl- bzw. Heteroarylgruppen, aus denen das aromatische Ringsystem zusammengesetzt sind, sind bevorzugt ausgewählt aus der Gruppe bestehend aus Benzol, Naphthalin, Phenanthren, Triphenylen, Pyridin, Thiophen, Furan, Pyrrol, Carbazol, Fluoren, Benzofuran, Benzothiophen, Dibenzofuran, Dibenzothiophen, Chinolin, Isochinolin, Phenanthridin, Phenanthrolin, Azacarbazol, Imidazol und Benzimidazol, welche jeweils mit einem oder mehreren Resten R¹ substituiert sein können. Weiterhin bevorzugte Reste R sind aromatische bzw. heteroaromatische Aminogruppen, wobei die Aryl- bzw. Heteroarylgruppen durch einen oder mehrere Reste R¹ substituiert sein können.

Wenn die erfindungsgemäße Verbindung als Matrixmaterial für einen phosphoreszierenden Emitter eingesetzt wird, enthält bevorzugt keine der Aryl- oder Heteroarylgruppen, die das aromatische bzw. heteroaromatische Ringsystem bilden oder die in der aromatischen bzw. heteroaromatischen Aminogruppe vorliegen, mehr als zwei direkt aneinander kondensierte aromatische Sechsringe. Bevorzugt enthalten die Reste R überhaupt keine direkt aneinander kondensierten aromatischen Sechsringe. Unter einer Aryl- oder Heteroarylgruppe mit nicht mehr als zwei direkt aneinander kondensierten aromatischen Sechsringen werden einfache Aryl- oder Heteroarylgruppen, wie zum Beispiel Benzol oder Pyridin, oder Aryl- oder Heteroarylgruppen mit genau zwei aneinander kondensierten aromatischen Sechsringen, wie zum Beispiel Naphthalin oder Chinolin verstanden. Weiterhin werden hierunter Heteroarylgruppen verstanden, in denen aromatische Fünfringe und Sechsringe, aber nicht aromatische Sechsringe direkt aneinander ankondensiert sind, wie zum Beispiel Carbazol, Dibenzofuran, Dibenzothiophen oder Benzimidazol. Weiterhin können auch Phenanthren oder Triphenylen geeignet sein.

Bevorzugte Gruppen R sind ausgewählt aus der Gruppe bestehend aus Benzol, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta- oder para-verknüpftes, lineares oder verzweigtes Quaterphenyl, Fluoren, insbesondere 1-, 2-, 3- oder 4-Fluoren, Spirobifluoren, insbesondere 1-, 2-, 3- oder 4-Spirobifluoren, 1-oder 2-Naphthalin, Pyrrol, Furan, Thiophen, Indol, Benzofuran, Benzothiophen, Carbazol, insbesondere 1-, 2-, 3- oder 4-Carbazol oder N-Carbazol, Dibenzothiophen, insbesondere 1-, 2-, 3- oder 4-Dibenzothiophen, Dibenzofuran, insbesondere 1-, 2-, 3- oder 4-Dibenzofuran, 1,3,5-Triazin, Pyridin, Pyrimidin, Pyrazin, Pyridazin, Indenocarbazol, verbrücktes Carbazol, Indolocarbazol, Phenanthren, Triphenylen oder Kombinationen aus zwei oder drei dieser Gruppen. Dabei können diese Gruppen jeweils durch einen oder mehrere Reste R¹ substituiert sein.

Wenn R für ein aromatisches bzw. heteroaromatisches Ringsystem steht, dann ist dieses bevorzugt ausgewählt aus den Gruppen der folgenden Formeln (R-1) bis (R-33), 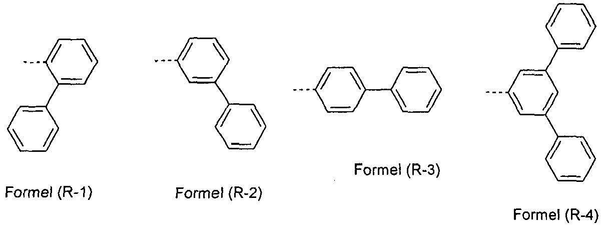 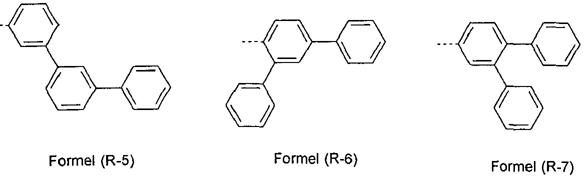 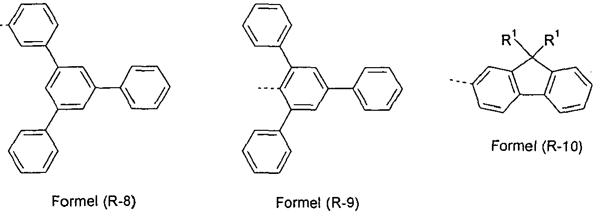 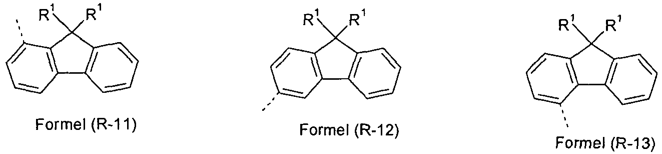 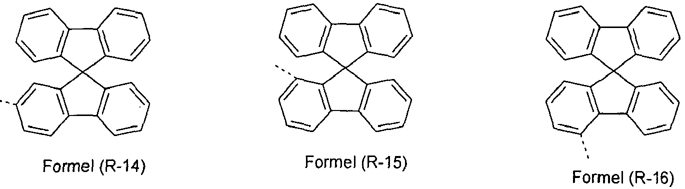 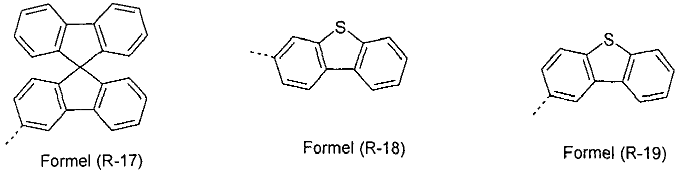 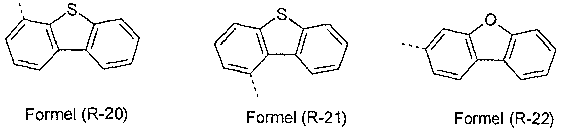 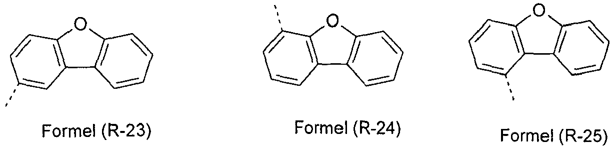 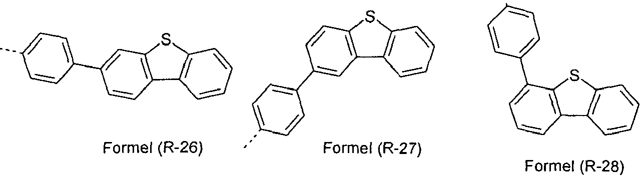 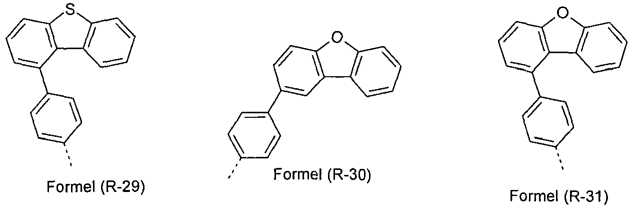 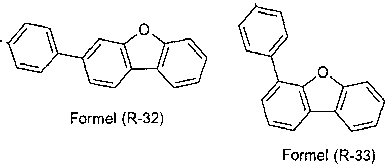 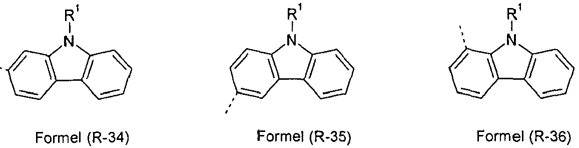 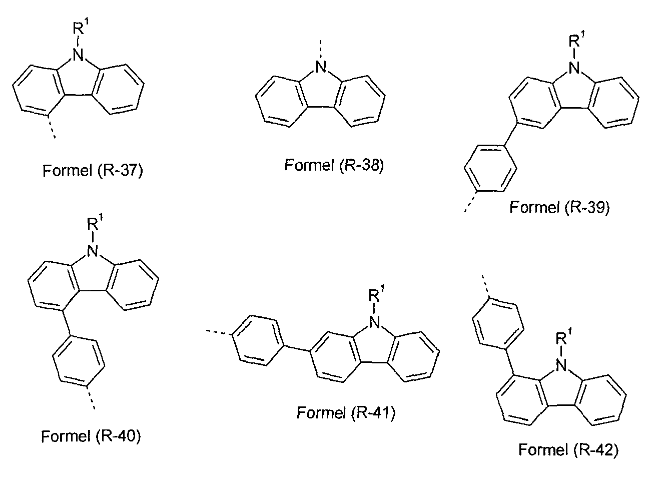 wobei die gestrichelte Bindung die Bindung an das Grundgerüst andeutet und die Gruppen jeweils durch einen oder mehrere Reste R¹ substituiert sein können, bevorzugt aber unsubstituiert sind.

Wenn die erfindungsgemäßen Verbindungen als Elektronentransportmaterial verwendet werden, ist es bevorzugt, wenn mindestens eine der Gruppen R und/oder L für ein elektronenarmes heteroaromatisches Ringsystem oder -C(=O)Ar¹ oder -P(=O)(Ar¹)₂ steht. Elektronenarme Heteroaromaten sind erfindungsgemäß Fünfringheteroaromaten mit mindestens zwei Heteroatomen oder Sechsringheteroaromaten, an die jeweils noch ein oder mehrere aromatische oder heteroaromatische Gruppen ankondensiert sein können, zum Beispiel substituierte oder unsubstituierte Imidazole, Pyrazole, Thiazole, Oxazole, Oxadiazole, Triazole, Pyridine, Pyrazine, Pyrimidine, Pyridazine, Triazine, Benzimidazole, etc., insbesondere solche, wie sie im Folgenden aufgeführt sind. Bevorzugte Gruppen R und/oder L sind weiterhin auch substituierte oder unsubstituierte kondensierte Arylgruppen, insbesondere Naphthalin, Anthracen, Pyren, Phenanthren, Triphenylen und Benzanthracen.

Wenn die erfindungsgemäße Verbindung bzw. die oben aufgeführten bevorzugten Verbindungen als Matrixmaterial für einen phosphoreszierenden Emitter oder als Elektronentransportmaterial eingesetzt wird, ist es weiterhin bevorzugt, wenn mindestens eine Gruppe R eine einfache aromatische Gruppe oder eine elektronenarme Gruppe darstellt, insbesondere ausgewählt aus Strukturen gemäß den folgenden Formeln (13) bis (16) für R oder den Formeln (17), (18) oder (19) für L, 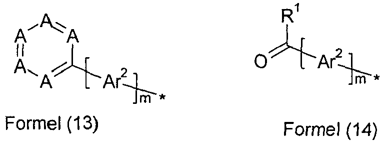 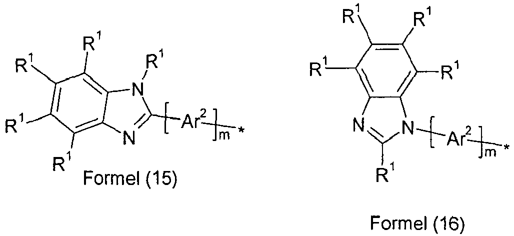 und/oder mindestens eine bivalente bzw. trivalente Gruppe L steht bevorzugt für eine Gruppe der folgenden Formeln (17) bis (19), 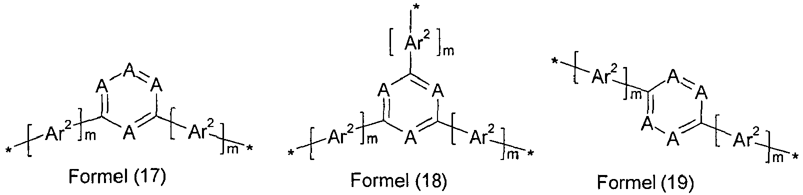 wobei R¹ die oben genannte Bedeutung hat, * die Position der Bindung der Gruppe gemäß Formel (13) bis (19) andeutet und weiterhin gilt:
A ist bei jedem Auftreten gleich oder verschieden CR¹ oder N, mit der Maßgabe, dass keine, eine, zwei oder drei Gruppen A für N stehen;
Ar² ist gleich oder verschieden bei jedem Auftreten ein bivalentes aromatisches oder heteroaromatisches Ringsystem mit 5 bis 16 C-Atomen, welches durch einen oder mehrere Reste R¹ substituiert sein kann;
m ist bei jedem Auftreten gleich oder verschieden 0 oder 1.

Als Matrixmaterial für phosphoreszierende Emitter sind weiterhin auch generell die oben aufgeführten aromatischen und heteroaromatischen Ringsysteme bevorzugt, die allgemein als bevorzugte Reste R genannt sind.

In einer besonders bevorzugten Ausführungsform der Erfindung steht mindestens ein Substituent R für eine Gruppe der oben genannten Formel (R-1) bis (R-17) oder Formel (13), und/oder mindestens eine Gruppe L steht für eine Gruppe der oben genannten Formeln (17) bis (19), wobei jeweils zwei oder drei Symbole A für N stehen und die anderen Symbole A für CR¹ stehen. Besonders bevorzugte Gruppen R sind daher die Gruppen der folgenden Formeln (20) bis (26), und besonders bevorzugte Gruppen L sind die Gruppen der folgenden Formeln (27) bis (34), 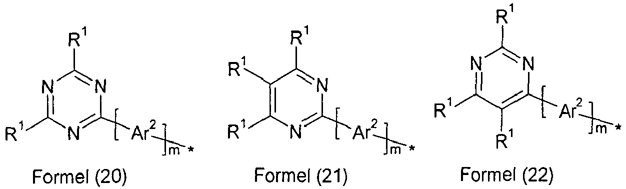 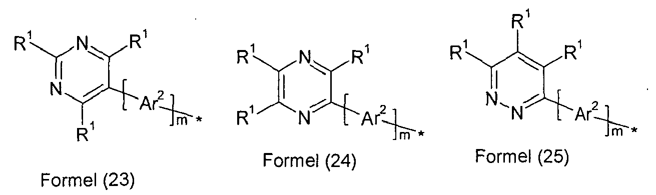 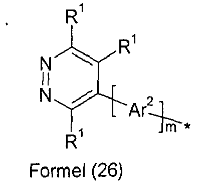 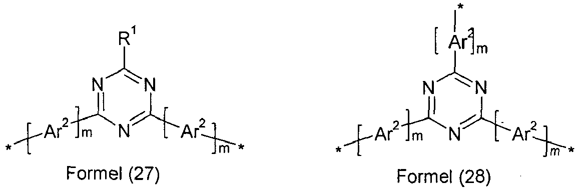 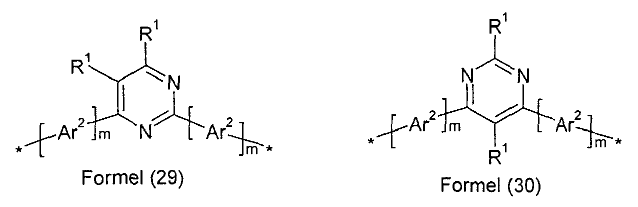 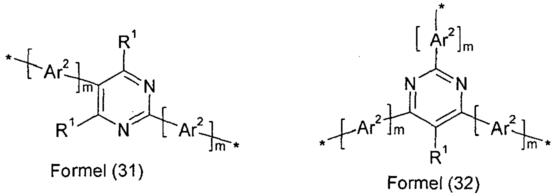 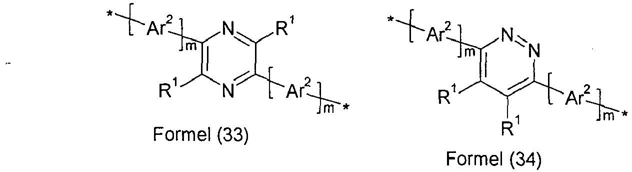 wobei die verwendeten Symbole und Indizes die oben genannten Bedeutungen aufweisen.

Wenn R für eine Gruppe der Formel (20) steht, dann steht R¹ in dieser Gruppe bevorzugt für ein aromatisches oder heteroaromatisches Ringsystem mit 5 bis 24 aromatischen Ringatomen, welches durch eine oder mehrere Alkylgruppen mit 1 bis 10 C-Atomen substituiert sein kann, insbesondere für Phenyl, Biphenyl, insbesondere ortho-, meta- oder para-Biphenyl, Terphenyl, insbesondere ortho-, meta-, para- oder verzweigtes Terphenyl, oder Quaterphenyl, insbesondere ortho-, meta-, para- oder verzweigtes Quaterphenyl.

Wenn R für eine Gruppe der Formel (21) bis (34) steht, dann steht R¹ in diesen Gruppen bevorzugt gleich oder verschieden bei jedem Auftreten für H, D oder ein aromatisches oder heteroaromatisches Ringsystem mit 5 bis 24 aromatischen Ringatomen, welches durch eine oder mehrere Alkylgruppen mit 1 bis 10 C-Atomen substituiert sein kann, insbesondere für H oder Phenyl, Biphenyl, insbesondere ortho-, meta- oder para-Biphenyl, Terphenyl, insbesondere ortho-, meta-, para- oder verzweigtes Terphenyl, oder Quaterphenyl, insbesondere ortho-, meta-, para- oder verzweigtes Quaterphenyl.

Wenn die erfindungsgemäße Verbindung als Matrixmaterial für einen phosphoreszierenden Emitter eingesetzt wird, kann es weiterhin bevorzugt sein, wenn mindestens ein Substituent R ausgewählt ist aus der Gruppe bestehend aus Triaryl- bzw. Heteroarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R¹ substituiert sein können, oder mindestens ein Substituent R steht für -N(Ar¹)₂. Diese Gruppen sind bevorzugt ausgewählt aus den Gruppen der folgenden Formeln (35) bis (59), 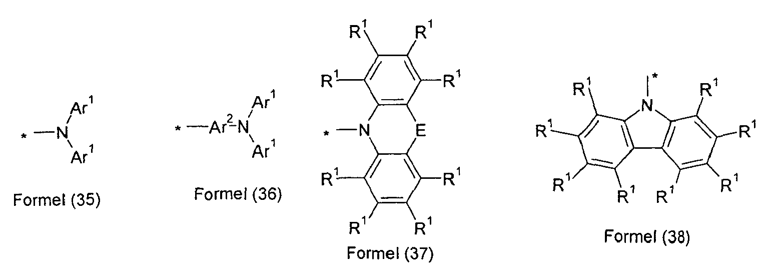 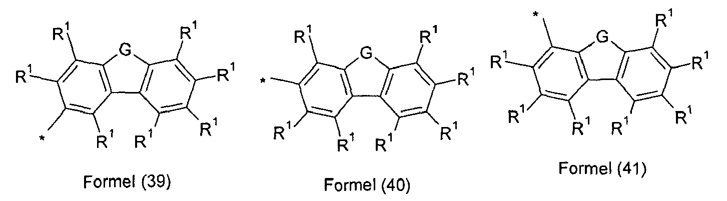 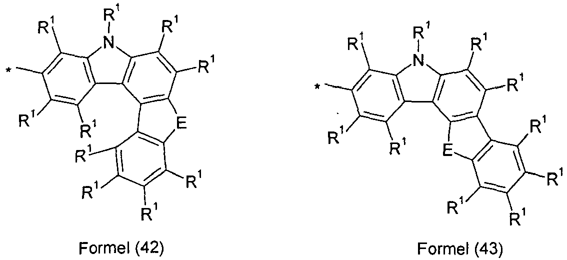 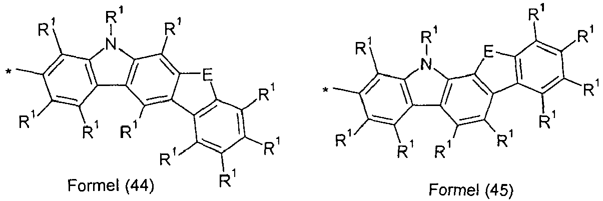 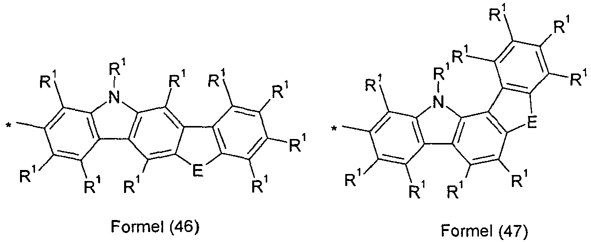 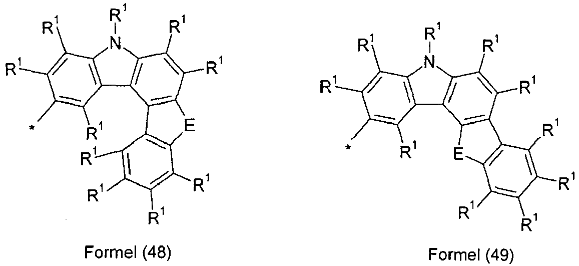 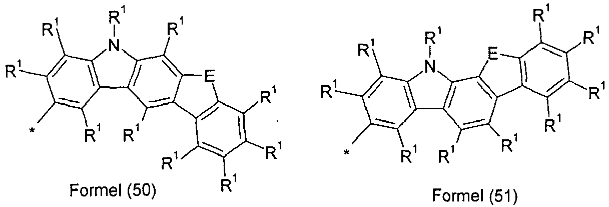 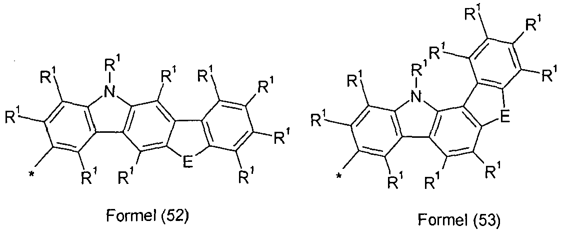 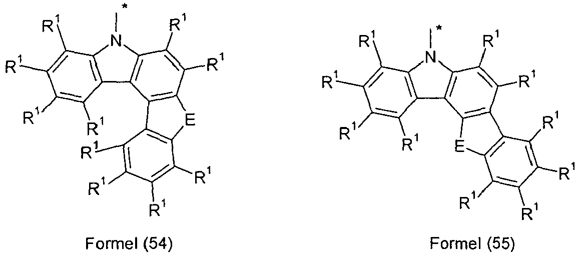 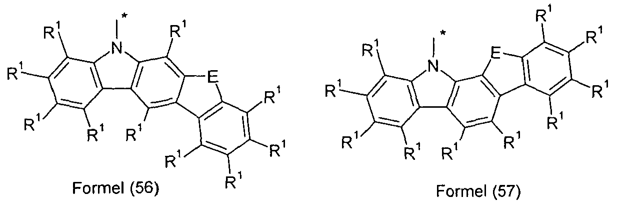 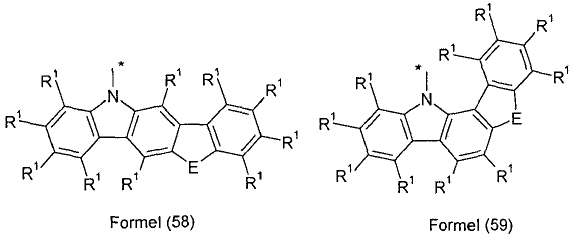 wobei * die Position der Bindung der Gruppe andeutet, die verwendeten Symbole die oben genannten Bedeutungen aufweisen und weiterhin gilt:
E ist ausgewählt aus der Gruppe bestehend aus C(R¹)₂, NR¹, O oder S;
G ist ausgewählt aus der Gruppe bestehend aus NR¹, O oder S.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formel (1) bzw. die bevorzugten Ausführungsformen als Matrixmaterial für einen fluoreszierenden Emitter oder als fluoreszierender Emitter verwendet werden, ist es bevorzugt, wenn mindestens einer der Reste R eine Gruppe enthält, die ausgewählt ist aus Naphthalin, Anthracen, Phenanthren, Pyren und/oder Benzanthracen, welches jeweils noch durch eine oder mehrere Gruppen R¹ substituiert sein kann.

In einer bevorzugten Ausführungsform der Erfindung sind die Reste R in den erfindungsgemäßen Verbindungen, die nicht für die oben genannten Gruppen stehen, gleich oder verschieden bei jedem Auftreten ausgewählt aus der Gruppe bestehend aus H, D, F, CN, N(Ar²)₂, C(=O)Ar², einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können oder einem aromatischen oder heteroaromatischen Ringsystem mit 5 bis 30 aromatischen Ringatomen, das jeweils mit einem oder mehreren Resten R² substituiert sein kann. Besonders bevorzugt ist R gleich oder verschieden bei jedem Auftreten ausgewählt aus der Gruppe bestehend aus H, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, oder einem aromatischen oder heteroaromatischen Ringsystem mit 5 bis 18 aromatischen Ringatomen, das jeweils mit einem oder mehreren Resten R² substituiert sein kann.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgrupp'en mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer besonders bevorzugten Ausführungsform der Erfindung treten die oben genannten Bevorzugungen gleichzeitig auf.

Beispiele für bevorzugte erfindungsgemäße Verbindungen sind die in der folgenden Tabelle abgebildeten Verbindungen.

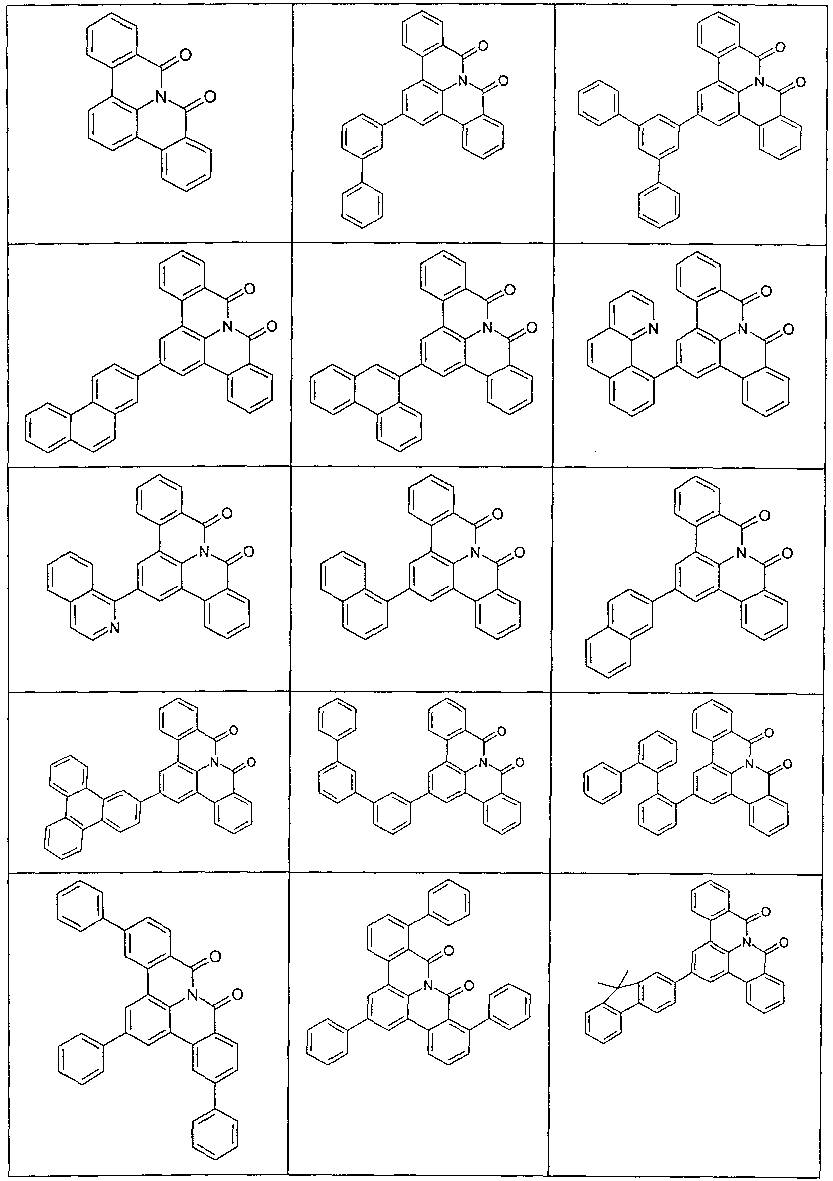

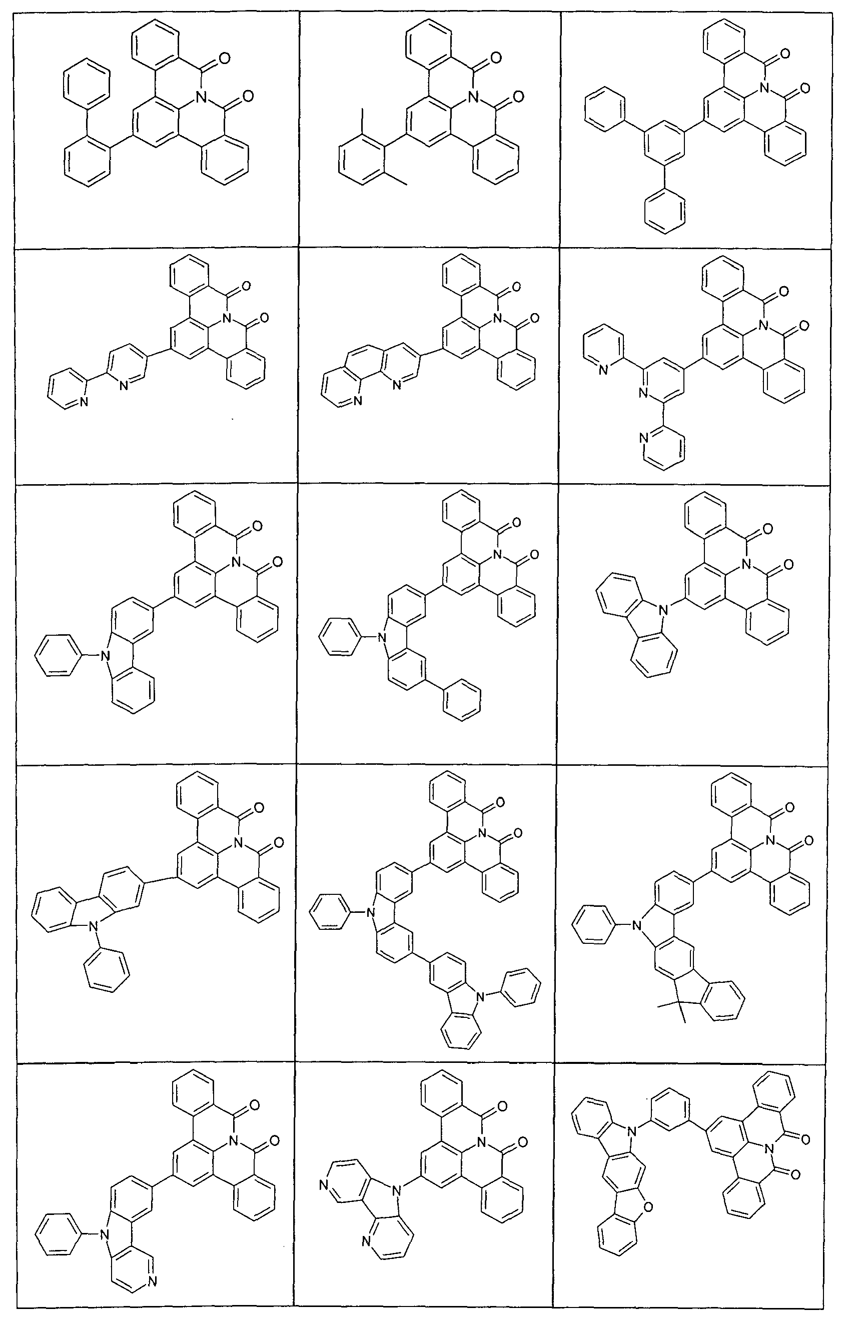

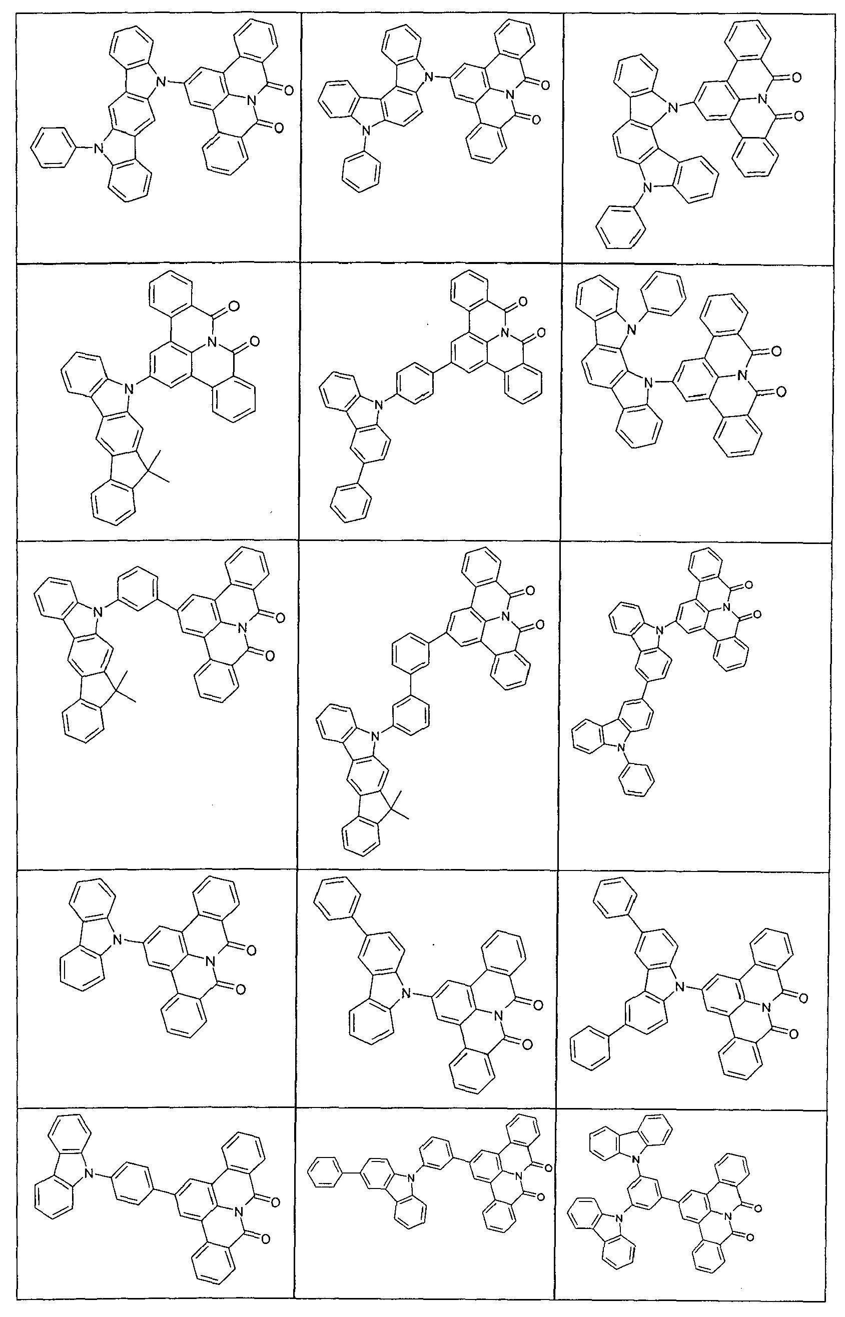

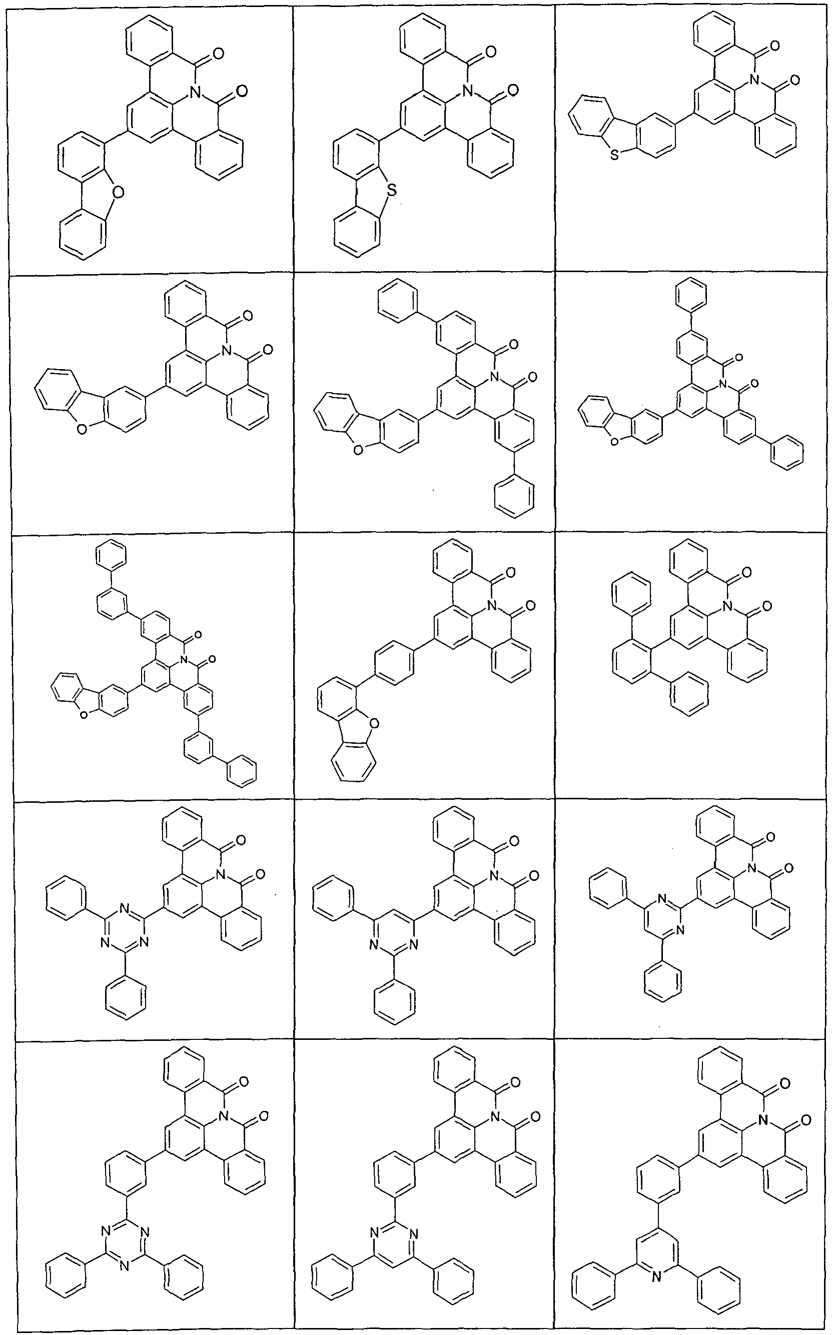

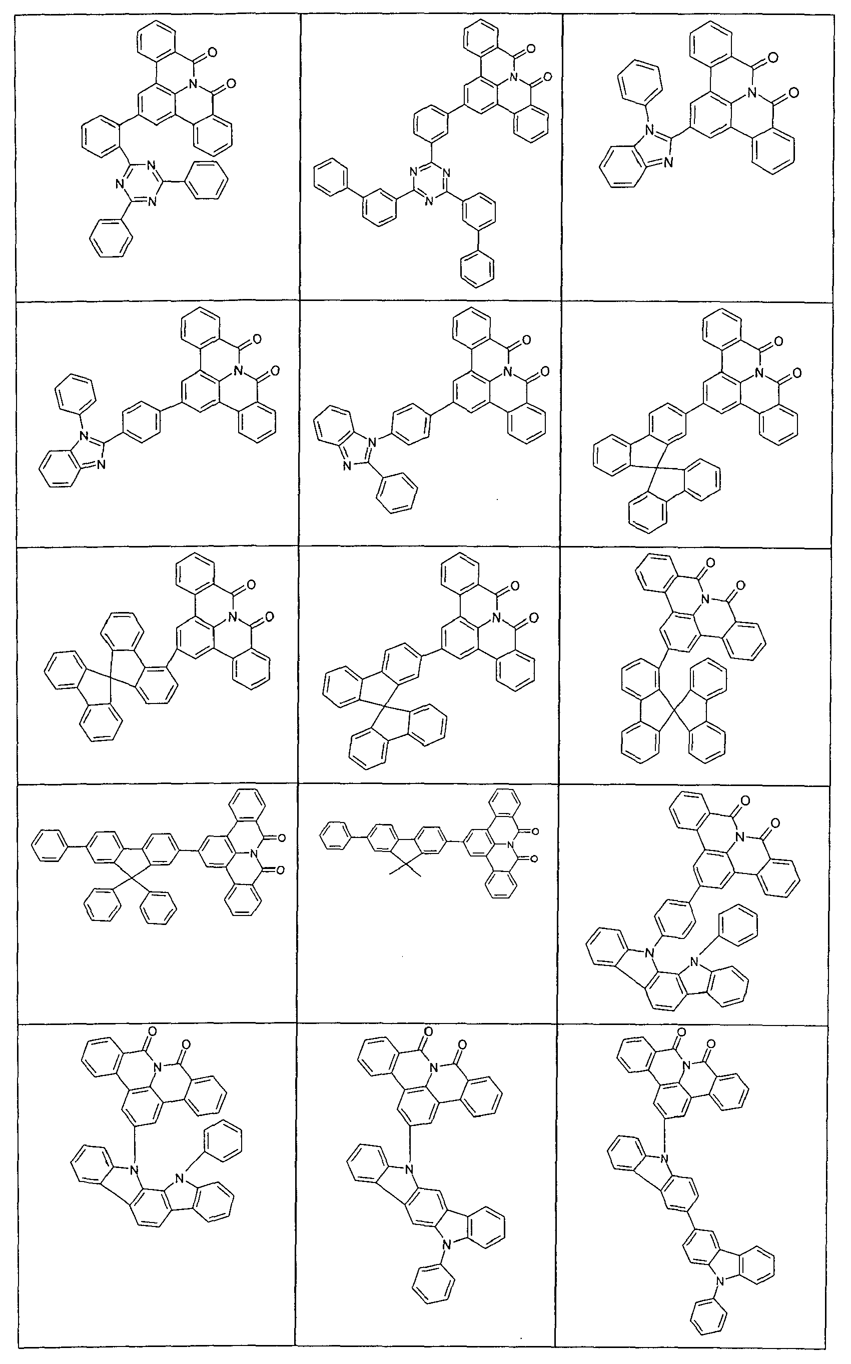

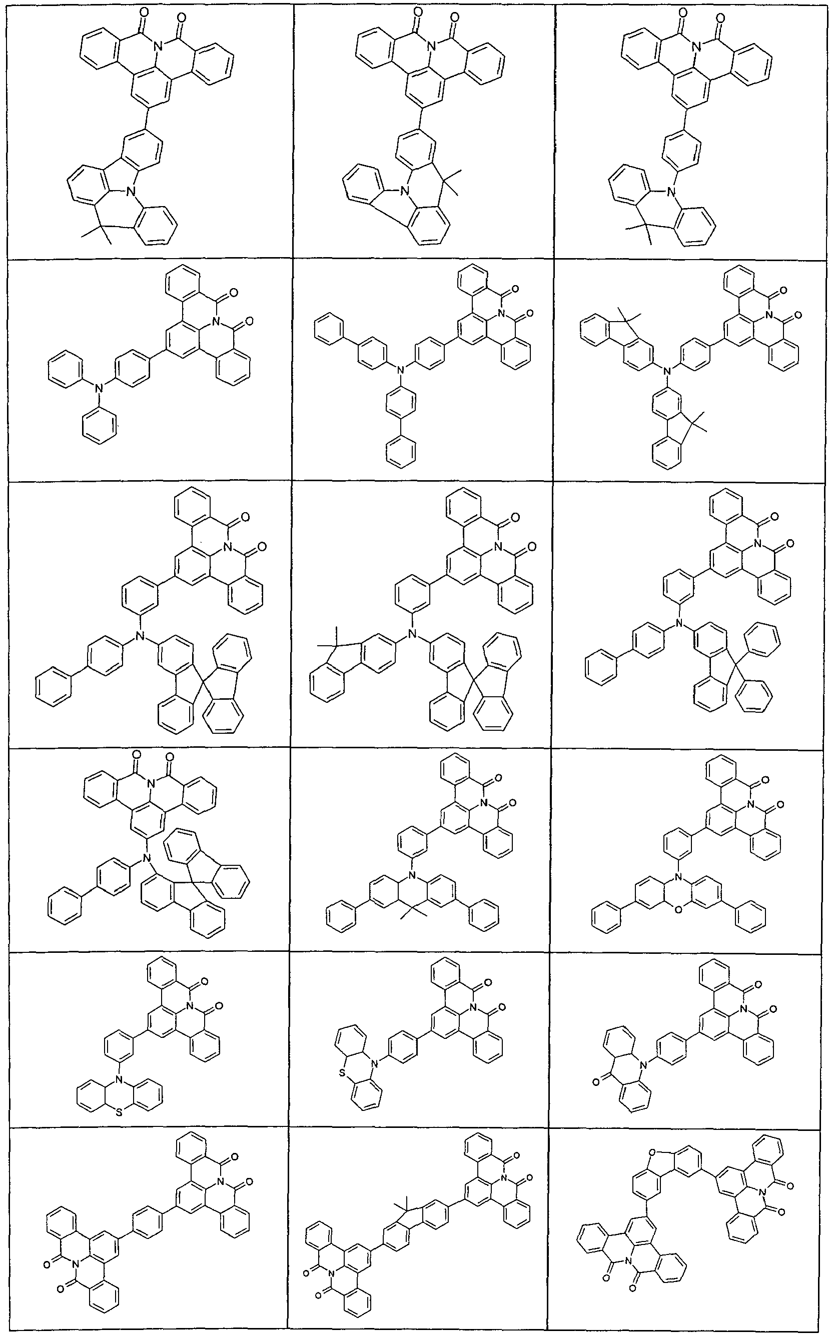

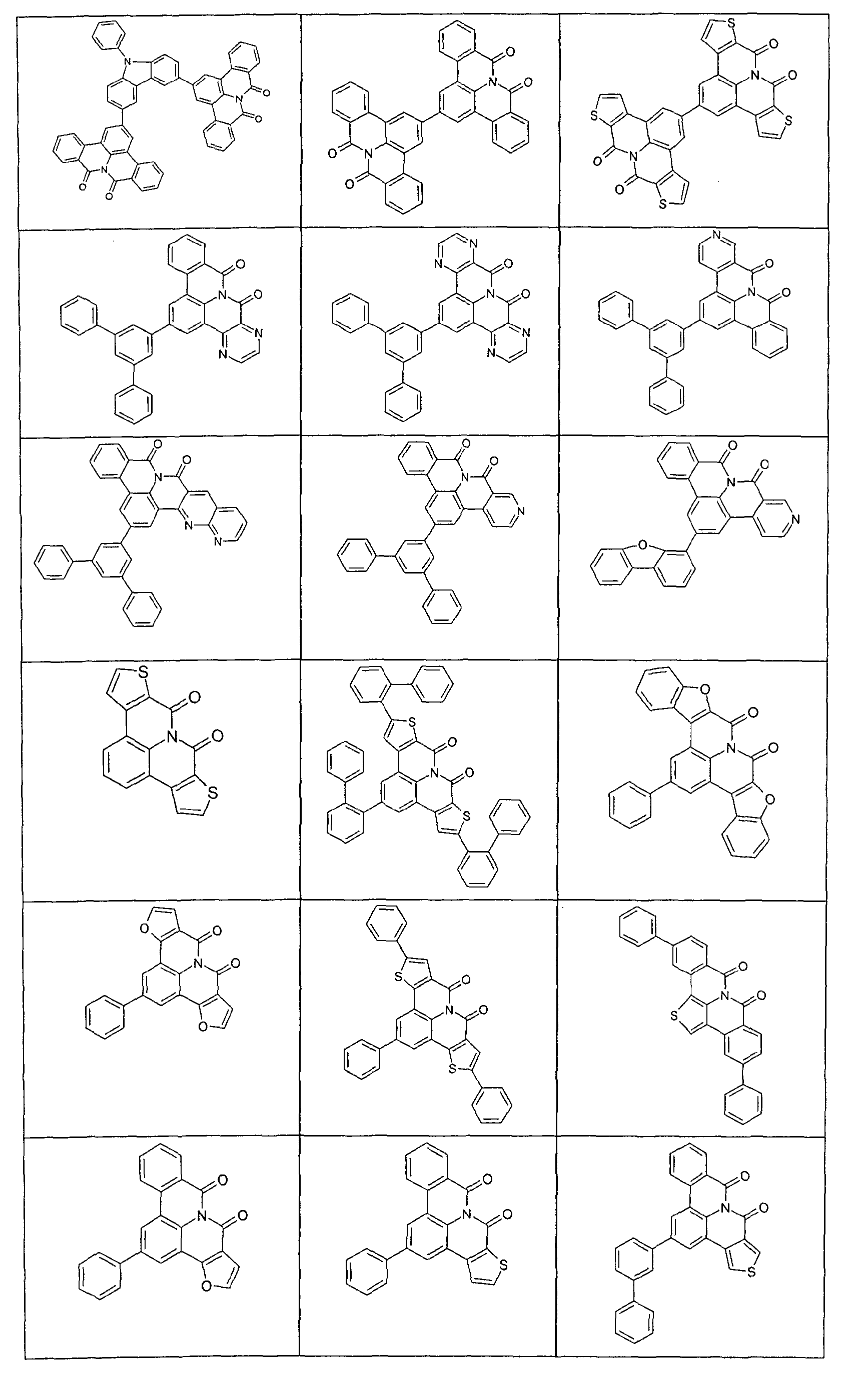

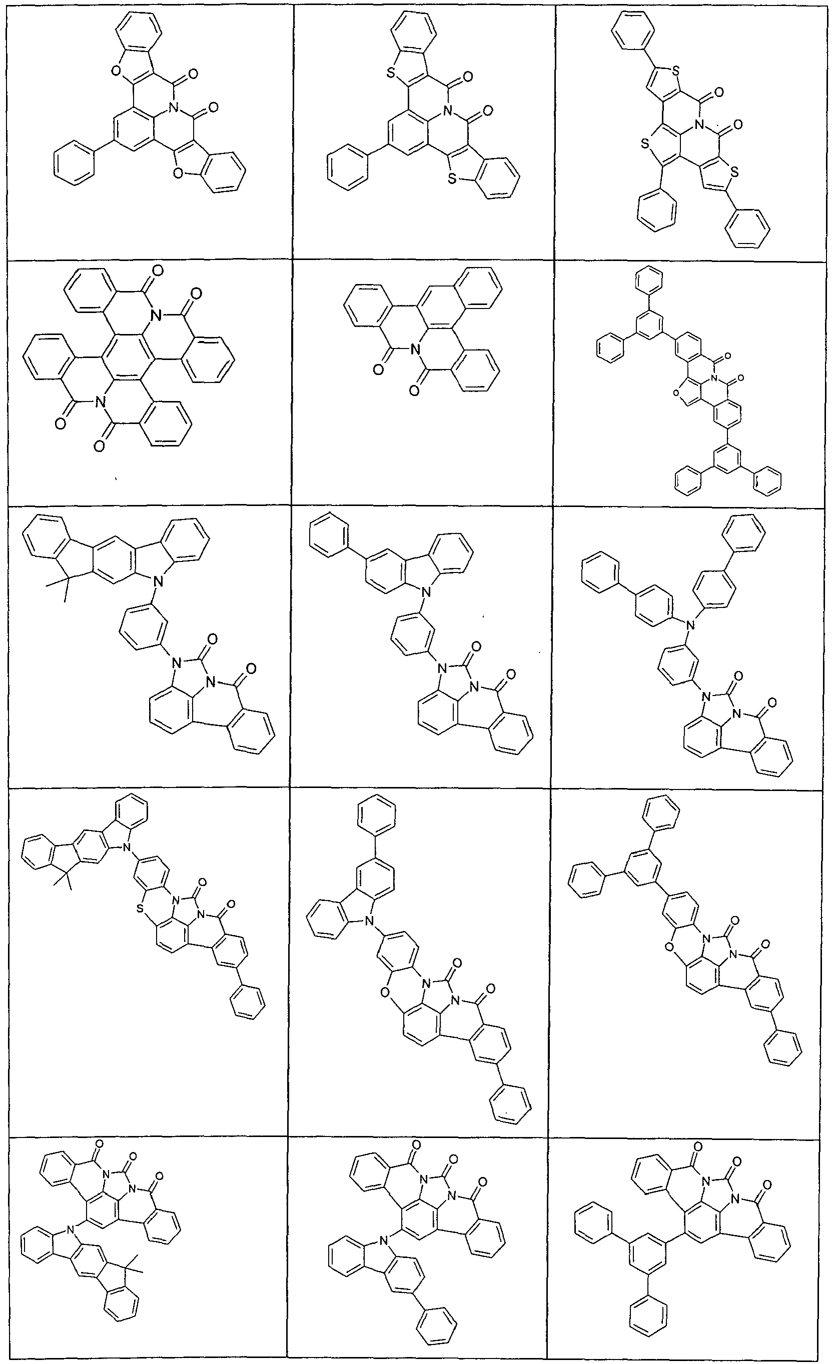

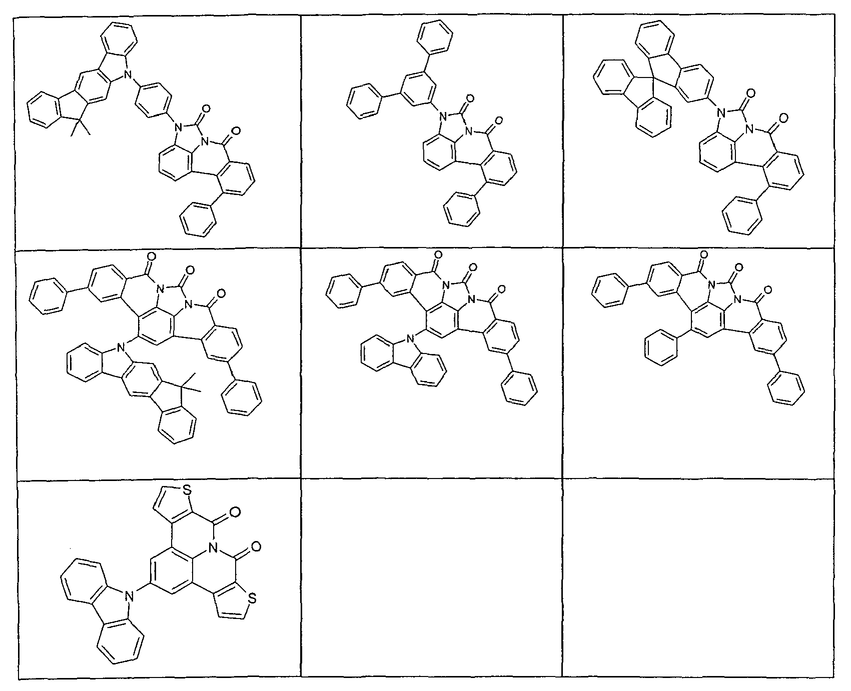

Die erfindungsgemäßen Verbindungen können nach dem Fachmann prinzipiell bekannten Syntheseschritten dargestellt werden, wie in Schema 1 und 2 schematisch dargestellt.

Wie in Schema 1 dargestellt, erhält man nach einer Schmidt-Reaktion und anschließender Reduktion das entsprechende 5,6-Dihydrophenanthridin-Derivat **(a)**, das unter Verwendung von in ortho-Stellung Halogen-substituiertem Carbonsäurehalogenid und anschließender Palladium-katalysierter intramolekularer Cyclisierung zum 8a-Aza-benzo[fg]naphthacen-8,9-dion umgesetzt wird. Durch Halogenierung, beispielsweise Bromierung mit NBS, kann dieser Grundbaustein halogeniert werden. In einer Folgereaktion kann beispielsweise mit einer Suzuki-Kupplung, einer Buchwald- oder Ullmann-Reaktion zum gewünschten Derivat umgesetzt werden.

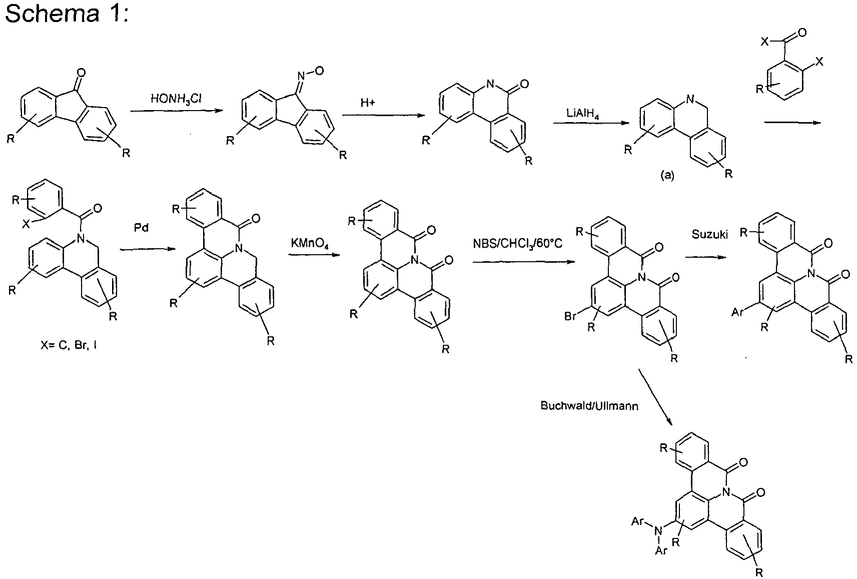

Alternativ kann an Stelle des in ortho-Position Halogen-substituierten Carbonsäurehalogenids ein ortho-substituiertes Halogen-benzylhalogenid eingesetzt werden, wie in Schema 2 dargestellt.

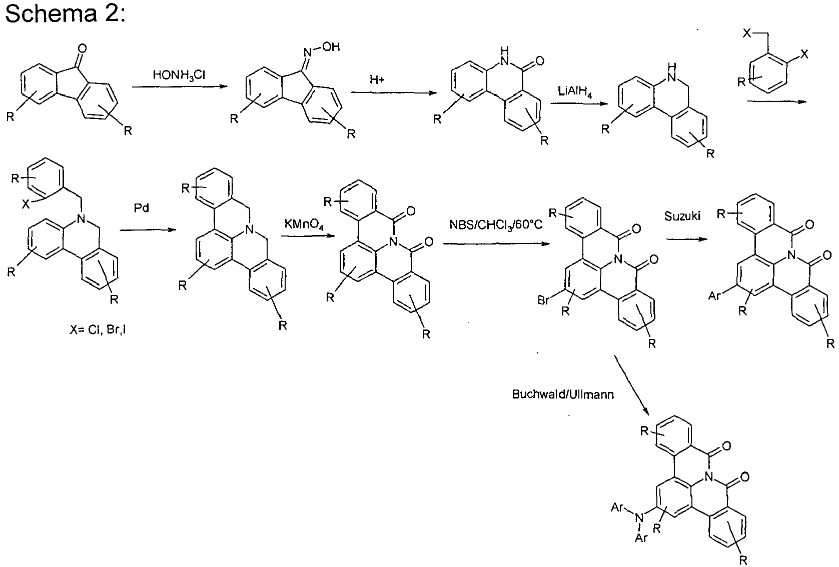

Ein weiterer Gegenstand der vorliegenden Erfindung ist ein Verfahren zur Herstellung einer Verbindung gemäß Formel (1), umfassend die Schritte:
a) Herstellung des halogenierten Grundgerüsts der Verbindung der Formel (1), wobei das Halogen bevorzugt Chlor, Brom oder Iod ist; und
b) Einführung mindestens eines Substituenten R in der Position des Halogens.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-lsopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise eine emittierende Verbindung, insbesondere eine phosphoreszierende Verbindung.

Ein weiterer Gegenstand der vorliegenden Erfindung ist die Verwendung der erfindungsgemäßen Verbindungen in einer elektronischen Vorrichtung, insbesondere in einer organischen Elektrolumineszenzvorrichtung.

Die organischen Elektrolumineszenzvorrichtungen und die lichtemittierenden elektrochemischen Zellen können für verschiedene Anwendungen eingesetzt werden, beispielsweise für einfarbige oder mehrfarbige Displays, für Beleuchtungsanwendungen oder für medizinische und/oder kosmetische Anwendungen, beispielsweise in der Phototherapie.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoffsensibilisierten Solarzellen (O-DSSC), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs) und besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Intetlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Weiterhin lässt sich weiße Emission bevorzugt erzeugen durch Verwendung einer blauen Emissionsschicht und einer Emissionsschicht, die rot und grün emittiert, wobei diese beiden Emissionsschichten durch eine Ladungserzeugungsschicht voneinander separiert sein können.

Die Verbindung gemäß Formel (1) kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution.

In einer weiteren Ausführungsform der Erfindung enthält die organische Elektrolumineszenzvorrichtung die Verbindung gemäß Formel (1) bzw. die oben ausgeführten bevorzugten Ausführungsformen in einer optischen Auskopplungsschicht. Unter einer optischen Auskopplungsschicht wird dabei eine Schicht verstanden, die nicht zwischen der Anode und der Kathode liegt, sondern die außerhalb der eigentlichen Vorrichtung auf eine Elektrode aufgebracht wird, beispielsweise zwischen einer Elektrode und einem Substrat, um die optische Auskopplung zu verbessern.

In einer bevorzugten Ausführungsform der Erfindung wird die erfindungsgemäße Verbindung als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine Verbindung gemäß Formel (1) als Matrixmaterial enthält.

Wenn die Verbindung gemäß Formel (1) bzw. die oben ausgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand verstanden. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) bzw. der oben ausgeführten bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. der oben ausgeführten bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Je nach Wahl des Matrixmaterials kann auch eine geringere Emitterkonzentration bevorzugt sein, wie z. B. in der nicht offen gelegten Anmeldung EP 11002816.4 beschrieben.

Eine weitere bevorzugte Ausführungsform der vorliegenden Erfindung ist der Einsatz der Verbindung gemäß Formel (1) bzw. der oben ausgeführten bevorzugten Ausführungsformen als Matrixmaterial für einen phosphoreszierenden Emitter in Kombination mit einem weiteren Matrixmaterial. Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Als phosphoreszierende Verbindung (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1) bzw. die oben ausgeführten bevorzugten Ausführungsformen als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die Verbindung gemäß Formel (1) bzw. die oben ausgeführten bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

Es ist weiterhin möglich, die Verbindung gemäß Formel (1) bzw. die oben ausgeführten bevorzugten Ausführungsformen sowohl in einer Lochblockierschicht bzw. Elektronentransportschicht als auch als Matrix in einer emittierenden Schicht zu verwenden.

In nochmals einer weiteren Ausführungsform der Erfindung wird die Verbindung gemäß Formel (1) bzw. die oben ausgeführten bevorzugten Ausführungsformen in einer Lochtransportschicht bzw. in einer Elektronenblockierschicht bzw. Exzitonenblockierschicht eingesetzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen rot oder grün phosphoreszierenden Emitter eingesetzt werden.
2. Die erfindungsgemäßen Verbindungen weisen eine hohe thermische Stabilität auf.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
4. Auch bei Verwendung als Elektronentransportmaterial führen die erfindungsgemäßen Verbindungen zu sehr guten Eigenschaften in Bezug auf die Effizienz, die Lebensdauer und die Betriebsspannung von organischen Elektrolumineszenzvorrichtungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Angaben bei den literaturbekannten Edukten sind die CAS-Nummern.

### Beispiel 1: 5,6-Dihydrophenanthridin

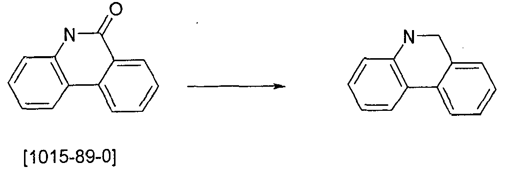

8.78 g (153 mmol) LiAlH₄ werden in 1000 mL THF unter Schutzgasatmosphäre vorgelegt. 30 g (153 mmol) 5H-Phenanthridin-6-on werden portionsweise zugegeben und anschließend 8 h unter Rückfluss erhitzt. Das Lösungsmittel wird im Vakuum entfernt und ohne weitere Aufbringung weiter eingesetzt. Die Ausbeute beträgt 27 g (97 %).

Analog werden folgende Verbindungen erhalten:

| **Bsp.** | **Edukt 1** | **Produkt** | **Ausbeute** |
|---|---|---|---|
| 1a | 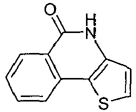 | 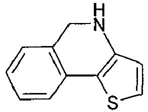 | 73% |
| | [1350243-25-2] | | |
| 1b | 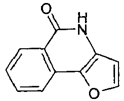 | 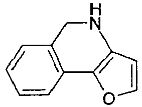 | 70% |
| | [630422-69-4 ] | | |
| 1c | 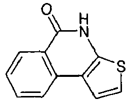 | 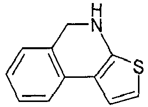 | 75% |
| | [420849-22-5 ] | | |

### Beispiel 2: 15-(2-Iod-benzoyl)-5H-phenanthridin-6-on

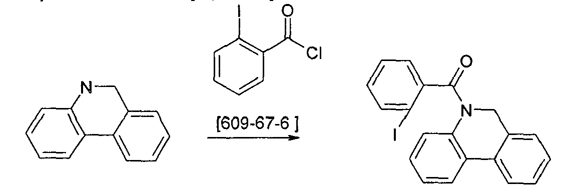

20 g (113 mmol) 5,6-Dihydrophenanthridin werden in 500 mL THF unter Schutzgasatmosphäre gelöst und auf -25 °C gekühlt. 49.9 g (113 mmol) 2-lodbenzoylchlorid werden in 300 mL THF gelöst und zu der Reaktionsmischung zugetropft, so dass die Temperatur -25 °C nicht übersteigt. Nach 1 h bei -25 °C lässt man langsam auf Raumtemperatur kommen und rührt dann 1 h bei Raumtemperatur. Nach dieser Zeit wird das Reaktionsgemisch auf Eis gegossen und dreimal mit Essigsäureethylester extrahiert. Die vereinigten organischen Phasen werden über Na₂SO₄ getrocknet und eingeengt. Der Rückstand wird aus n-Heptan umkristallisiert. Die Ausbeute beträgt 40 g (90 %).

Analog werden folgende Verbindungen erhalten:

| **Bsp.** | **Edukt 1** | **Edukt 2** | **Produkt** | **Ausbeute** |
|---|---|---|---|---|
| 2a | 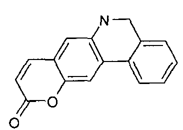 | 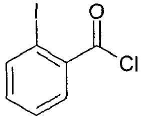 | 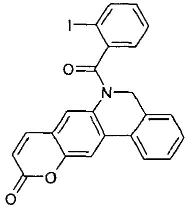 | 76% |
| | [1122399-53-4] | [609-67-6 ] | | |
| 2b | 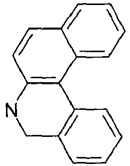 | 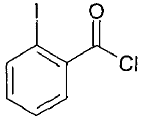 | 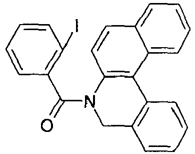 | 87% |
| | | [609-67-6] | | |
| | [928307-79-3] | | | |
| 2c | 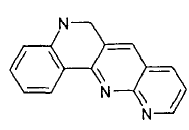 | 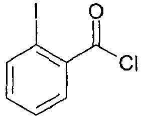 | 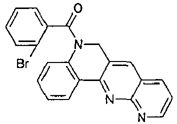 | 91 % |
| | [342404-76-6] | [609-67-6 ] | | |
| 2d | 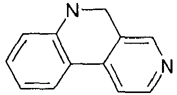 | 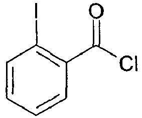 | 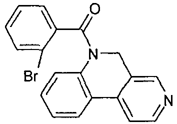 | 90% |
| | [61686-63-3] | [609-67-6 ] | | |
| 2e | 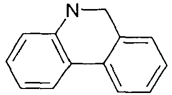 | 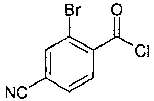 | 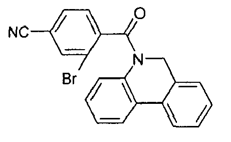 | 87% |
| | | [1261446-07-4] | | |
| 2f | 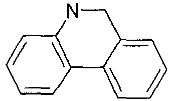 | 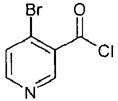 | 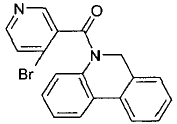 | 78% |
| | | [1367346-08-4] | | |
| 2g | 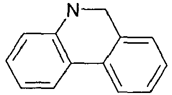 | 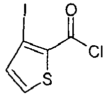 | 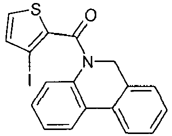 | 76% |
| | | [75427-00-8] | | |
| 2h | 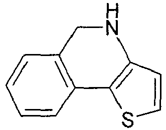 | 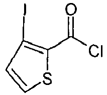 | 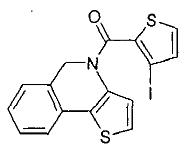 | 77% |
| | | [75427-00-8] | | |
| 2j | 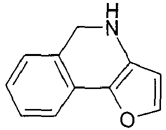 | 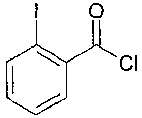 | 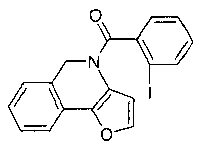 | 73% |
| | | [609-67-6 ] | | |

### Beispiel 3: 5-(2-Brom-benzyl)-5,6-dihydro-phenanthridin

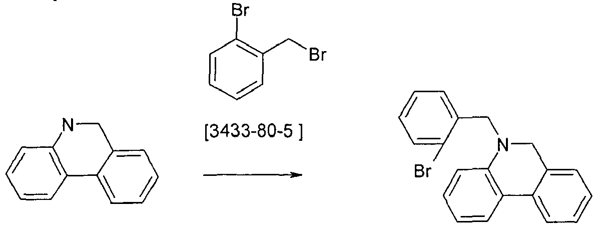

9.7 g (243 mmol) NaH 60%ig in Mineralöl werden in 500 mL Dimethylformamid unter Schutzgasatmosphäre gelöst. 43.9 g (243 mmol) 5,6-Dihydrophenanthridin werden in 500 mL DMF gelöst und zu der Reaktionsmischung zugetropft. Nach 1 h bei Raumtemperatur wird eine Lösung von 60.6 g (242 mmol) 2-Brombenzylbromid in 500 mL DMF zugetropft. Das Reaktionsgemisch wird dann 1 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Die Ausbeute beträgt 62 g (75 %).

Analog werden folgende Verbindungen erhalten:

| **Bsp.** | **Edukt 1** | **Edukt 2** | **Produkt** | **Ausbeute** |
|---|---|---|---|---|
| 3a | 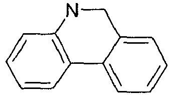 | 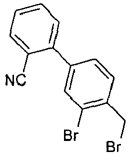 | 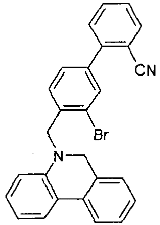 | 83% |
| | | [135689-85-9] | | |
| 3b | 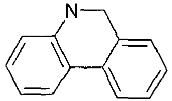 | 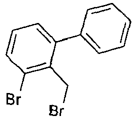 | 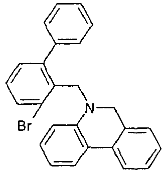 | 90% |
| | | [172976-02-2 ] | | |
| 3c | 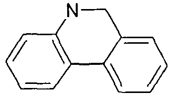 | 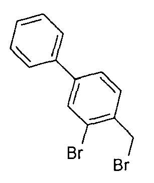 | 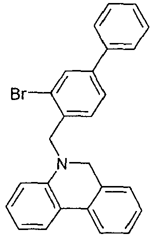 | 95% |
| | | [1396865-04-5] | | |

### Beispiel 4: 8a-Aza-benzo[fg]naphthacen-8,9-dion

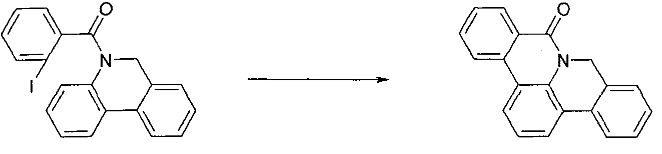

35 g (158 mmol) 5-(2-Brom-benzyl)-5H-phenanthridin-6-on werden in 1000 mL Dimethylformamid unter Schutzgasatmosphäre gelöst. Zu dieser Lösung werden 75.7 g (234 mmol) Tetrabutylammoniumbromid, 2.15 g (9.5 mmol) Palladiumacetat und 10 g (102 mmol) Kaliumacetat zugegeben. Anschließend wird das Gemisch bei 130 °C 2 h gerührt. Nach dieser Zeit wird das Reaktionsgemisch auf Raumtemperatur gekühlt. Der Rückstand wird abgesaugt und mit EtOH gewaschen. Der Rückstand wird aus n-Heptan/Toluol umkristallisiert. Die Ausbeute beträgt 17.7 g (74 %).

Analog werden folgende Verbindungen erhalten:

| **Bsp.** | **Edukt 1** | **Produkt** | **Ausbeute** |
|---|---|---|---|
| 4a | 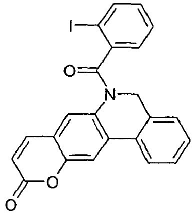 | 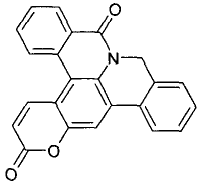 | 80% |
| 4b | 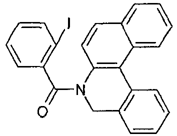 | 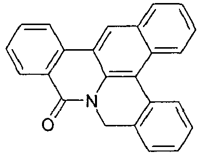 | 83 % |
| 4c | 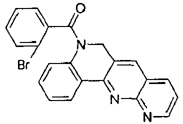 | 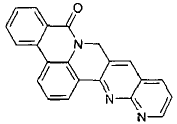 | 67 % |
| 4d | 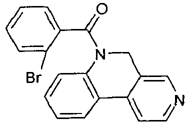 | 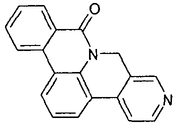 | 73% |
| 4e | 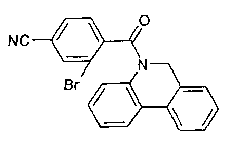 | 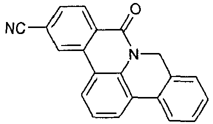 | 79% |
| 4f | 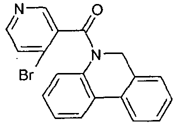 | 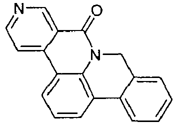 | 82% |
| 4g | 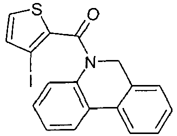 | 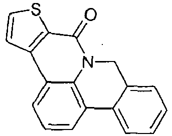 | 77% |
| 4h | 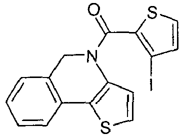 | 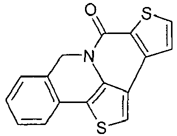 | 78% |
| 4j | 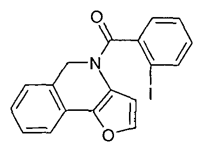 | 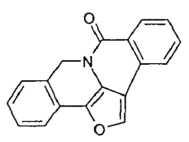 | 73% |

### Beispiel 5: 8H,9H-8a-Aza-benzo[fg]naphthacen

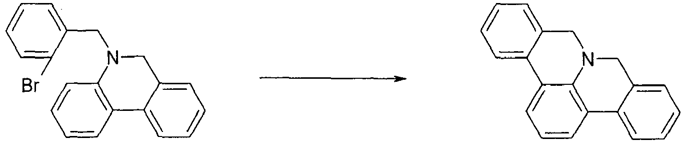

55.3 g (158 mmol) 5-(2-Brom-benzyl)-5,6-dihydrophenanthridin werden in 500 mL Dimethylformamid unter Schutzgasatmosphäre gelöst. Zu dieser Lösung werden 17.3 g (75 mmol) Benzyltrimethylammoniumbromid und 31.28 g (226 mmol) Kaliumcarbonat werden zugegeben. Anschließend wird unter Schutzgas 5.08 g (22 mmol) Pd(OAC)₂ zugegeben, und das Gemisch wird bei 120 °C 9 h gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird aus n-Heptan umkristallisiert. Die Ausbeute beträgt 34 g (81 %).

Analog werden folgende Verbindunaen erhalten:

| **Bsp.** | **Edukt 1** | **Produkt** | **Ausbeute** |
|---|---|---|---|
| 5a | 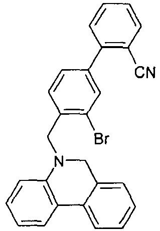 | 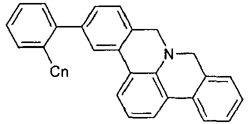 | 82% |
| 5b | 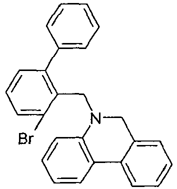 | 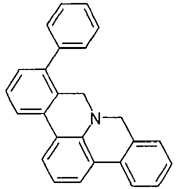 | 80% |
| 5c | 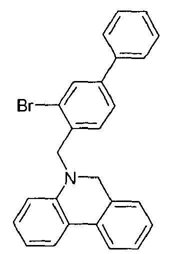 | 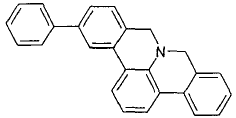 | 74% |

### Beispiel 6: 8a-Aza-benzo[fg]naphthacen-8,9-dion

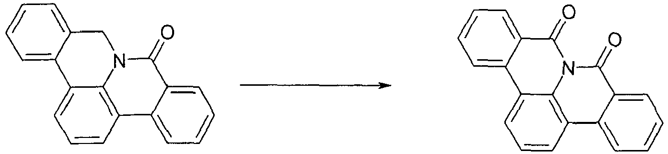

32 g (115 mmol) 9H-8a-Aza-benzo[fg]naphthacen-8-on werden in 1500 mL Aceton gelöst. Zu dieser Lösung werden 54.7 g (346 mmol) Kaliumpermanganat portionsweise werden zugegeben und zwei Tage bei Raumtemperatur gerührt. Nach dieser Zeit wird das restliche Kaliumpermanganat abfiltiert, die Lösung eingeengt und chromatographisch gereinigt (Laufmittel: Heptan/Dichloromethan, 5:1). Der Rückstand wird aus n-Heptan umkristallisiert. Die Ausbeute beträgt 24 g (73 %).

Analoa werden folgende Verbindungen erhalten:

| **Bsp.** | **Edukt 1** | **Produkt** | **Ausbeute** |
|---|---|---|---|
| 6a | 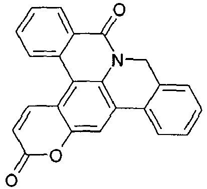 | 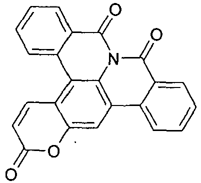 | 60% |
| 6b | 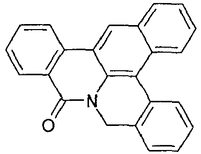 | 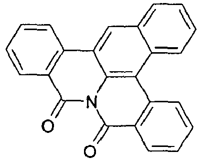 | 69% |
| 6c | 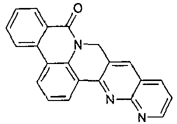 | 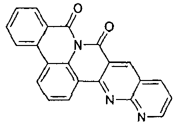 | 71% |
| 6d | 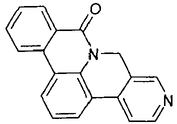 | 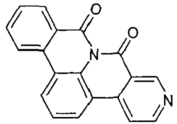 | 65% |
| 6e | 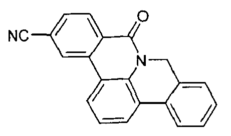 | 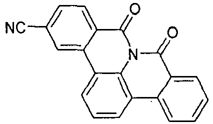 | 72% |
| 6f | 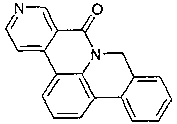 | 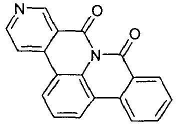 | 77% |
| 6g | 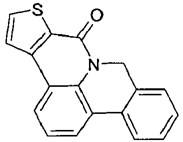 | 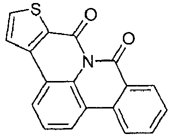 | 62% |
| 6h | 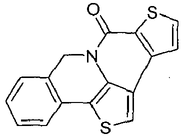 | 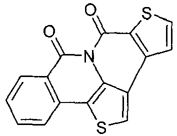 | 65% |
| 6j | 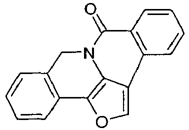 | 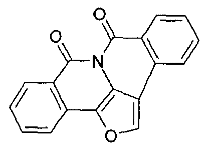 | 64% |

Analog werden folgende Verbindungen mit 6 eg. KMnO₄ erhalten:

| **Bsp.** | **Edukt 1** | **Produkt** | **Ausbeute** |
|---|---|---|---|
| 6i | 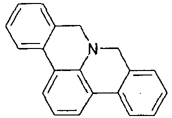 | 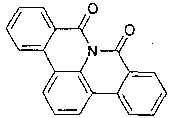 | 83% |
| 6k | 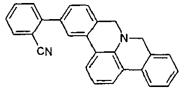 | 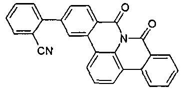 | 86% |
| 6l | 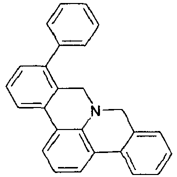 | 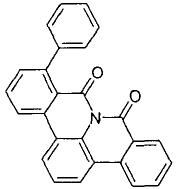 | . 79% |
| 6m | 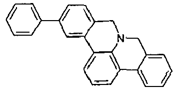 | 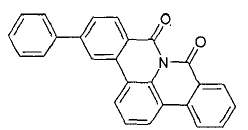 | 70% |

### Beispiel 7: 2-Brom-8a-aza-benzo[fg]naphthacen-8,9-dion

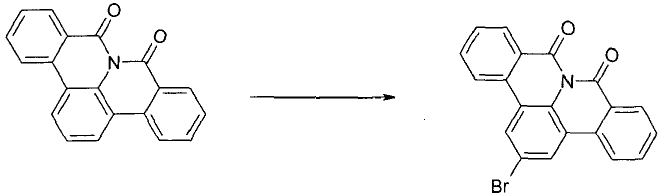

18.6 g (62,5mmol) 8a-Aza-benzo[fg]naphthacen-8,9-dion werden in 1800 mL CH₂Cl₂ vorgelegt. Anschließend gibt man 25.6 (312 mmol) Natriumacetat und 24.9 g (156 mmol) Brom zur Reaktionsmischung und rührt das Gemisch bei 80 °C für 30 h. Anschließend wird die Mischung mit 150 mL Wasser und 60 g NaOH-Plätzchen versetzt und der ausgefallene Feststoff abgesaugt. Das Produkt wird mit EtOH gewaschen und getrocknet. Ausbeute: 19 g (51 mmol), 81 % d. Th., Reinheit nach ¹H-NMR ca. 96 %.

Analoa werden folgende Verbindunaen erhalten:

| **Bsp.** | **Edukt 1** | **Produkt** | **Ausbeute** |
|---|---|---|---|
| 7a | 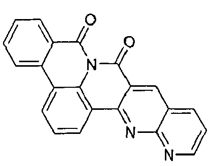 | 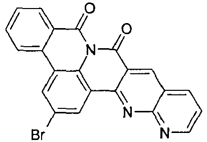 | 83 % |
| 7b | 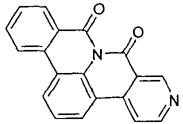 | 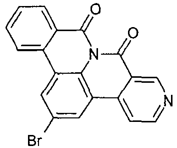 | 82 % |
| 7c | 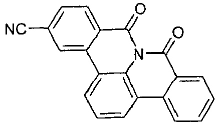 | 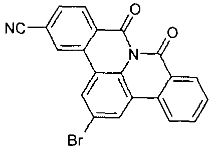 | 80% |
| 7d | 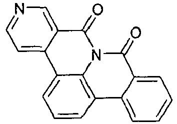 | 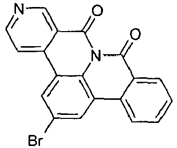 | 78 % |
| 7e | 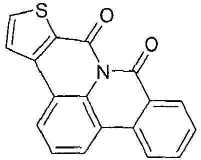 | 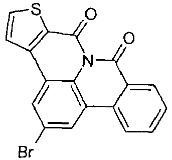 | 34% |
| 7f | 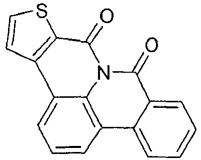 | 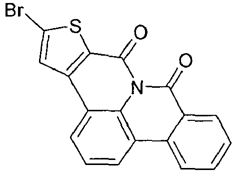 | 50% |
| 7h | 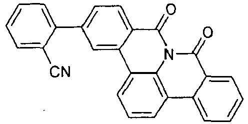 | 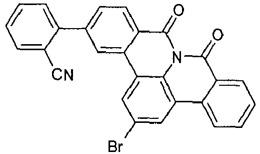 | 70% |
| 7j | 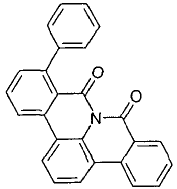 | 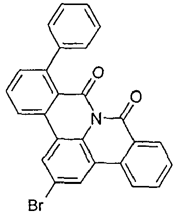 | 30% |
| 7i | 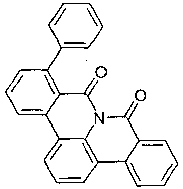 | 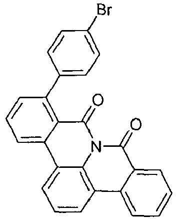 | 42% |
| 7k | 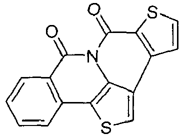 | 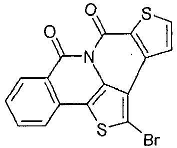 | 82% |
| 7l | 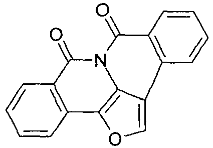 | 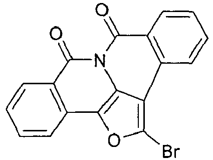 | 85% |
| 7m | 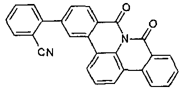 | 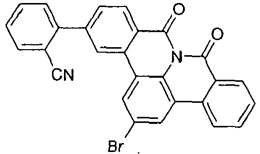 | 86% |
| 7n | 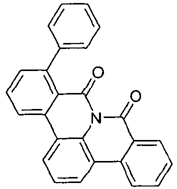 | 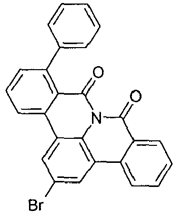 | 67% |
| 7o | 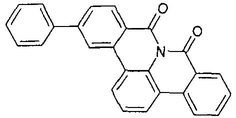 | 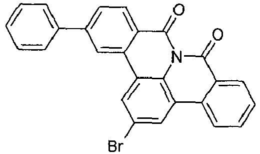 | 65% |

### Beispiel 8: 2-Dibenzofuran-4-yl-8a-aza-benzo[fg]naphthacen-8,9-dion

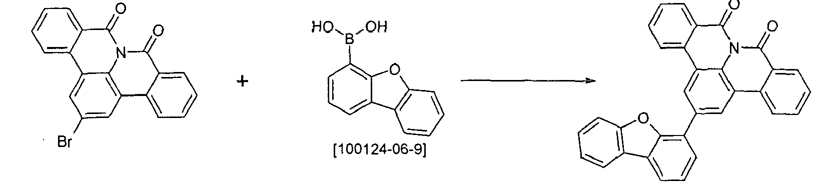

41.3 g (110.0 mmol) 4-dibenzofuranboronsäure, 38 g (110.0 mmol) 2-Brom-8a-aza-benzo[fg]naphthacen-8,9-dion und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Trio-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*Propanol umkristallisiert und abschließend im Hochvakuum sublimiert, Reinheit beträgt 99.9 %. Die Ausbeute beträgt 40 g (88 mmol), entsprechend 80% der Theorie.

Analog werden folgende Verbindungen erhalten:

| **Bsp.** | **Edukt 1** | **Edukt 2** | **Produkt** | **Ausbeute.** |
|---|---|---|---|---|
| 8a | 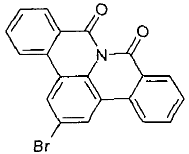 | 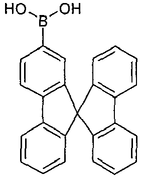 | 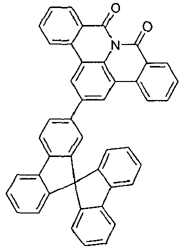 | 82% |
| | | 236389-21-2 | | |
| 8b | 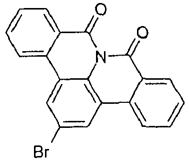 | 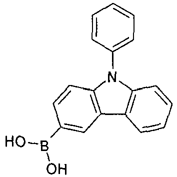 | 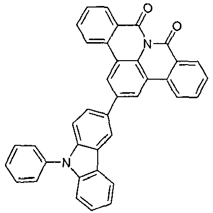 | 81% |
| | | [854952-58-2] | | |
| 8c | 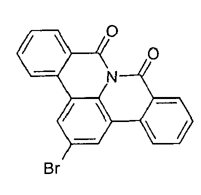 | 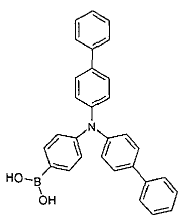 | 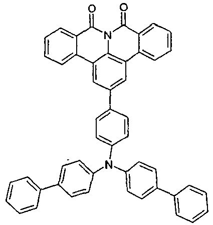 | 84% |
| | | [943836-24-6] | | |
| 8e | 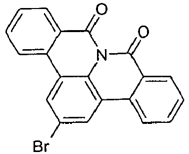 | 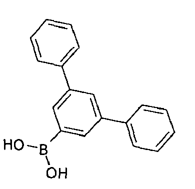 | 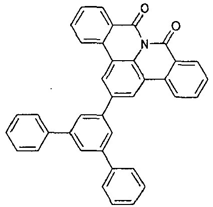 | 88% |
| | | [128388-54-5] | | |
| 8f | 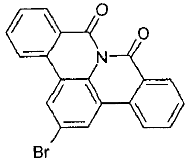 | 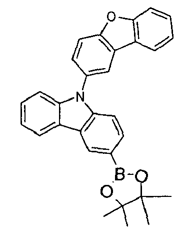 | 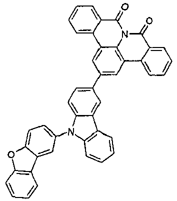 | 67% |
| | | [1338488-91-7] | | |
| 8g | 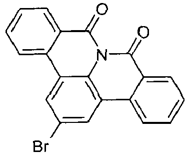 | 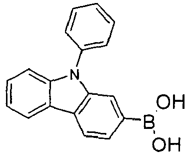 | 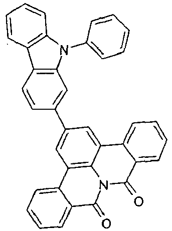 | 75% |
| | | [1001911-63-2] | | |
| 8h | 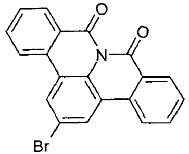 | 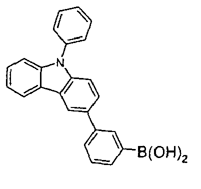 | 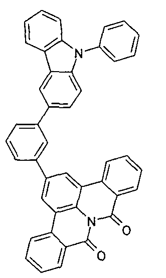 | 76% |
| | | [854952-60-6] | | |
| 8j | 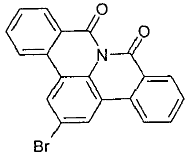 | 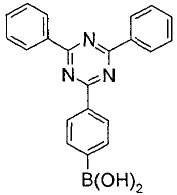 | 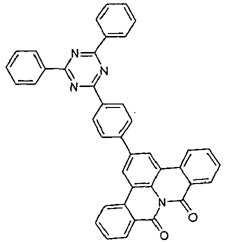 | 83% |
| | | [1313018-07-3] | | |
| 8i | 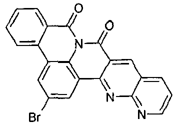 | 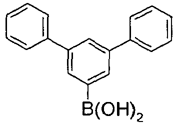 | 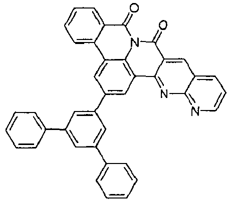 | 85% |
| | | [128388-54-5] | | |
| 8k | 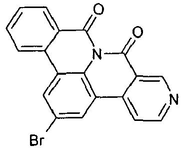 | 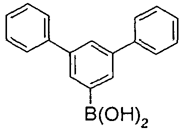 [128388-54-5] | 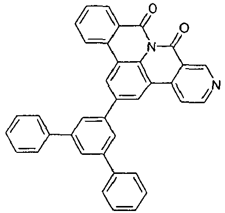 | 80% |
| 81 | 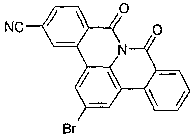 | 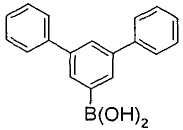 | 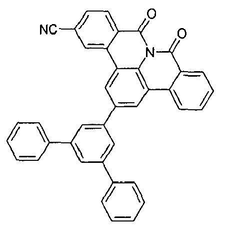 | 89% |
| | | [128388-54-5] | | |
| 8m | 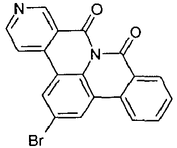 | 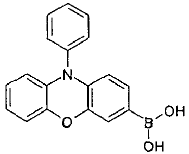 | 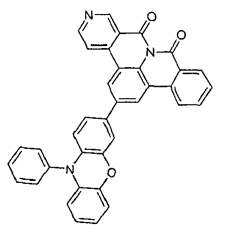 | 88% |
| | | [1314019-67-4] | | |
| 8n | 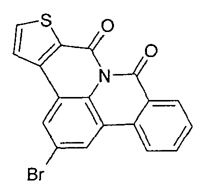 | 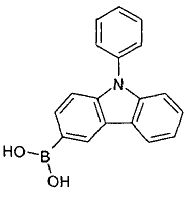 | 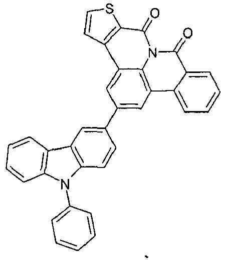 | 86% |
| | | [854952-58-2] | | |
| 8o | 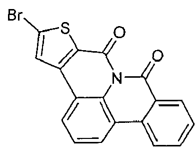 | 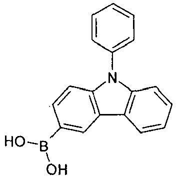 | 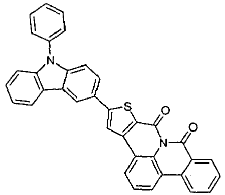 | 82% |
| | | [854952-58-2] | | |
| 8p | 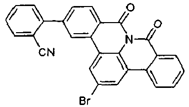 | 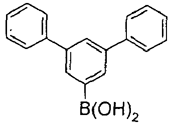 | 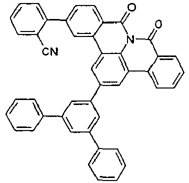 | 86% |
| | | [128388-54-5] | | |
| 8q | 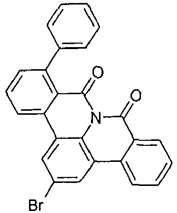 | 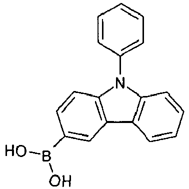 | 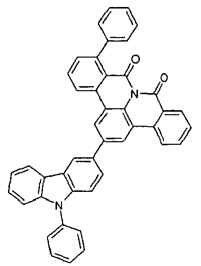 | 87% |
| | | [854952-58-2] | | |
| 8r | 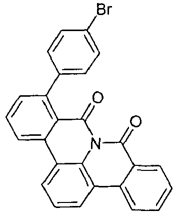 | 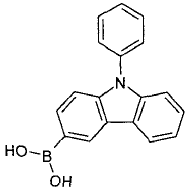 | 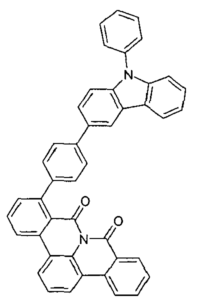 | 81% |
| | | [854952-58-2] | | |
| 8s | 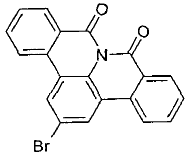 | 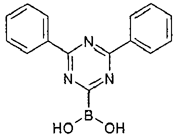 | 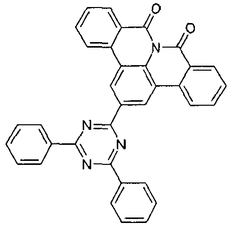 | 79% |
| | | [1251825-65-6] | | |
| 8t | 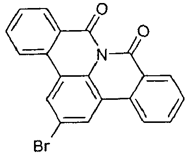 | 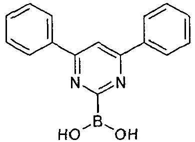 | 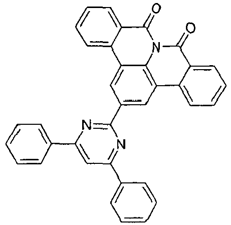 | 86% |
| | | [1314221-56-1] | | |
| 8u | 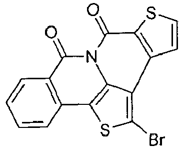 | 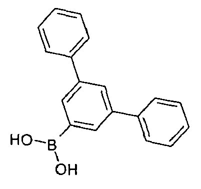 | 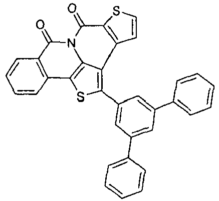 | 87% |
| | | [128388-54-5] | | |
| 8v | 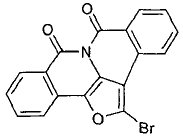 | 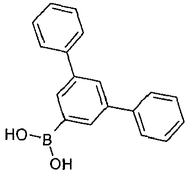 | 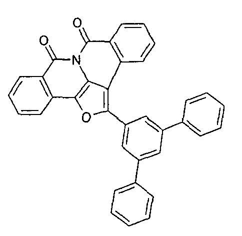 | 80% |
| | | [128388-54-5] | | |
| 8w | 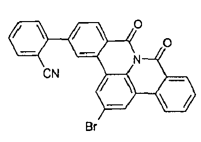 | 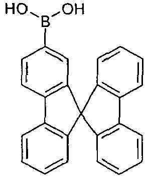 | 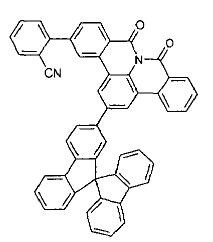 | 81% |
| | | 236389-21-2 | | |
| 8y | 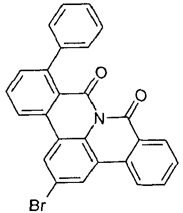 | 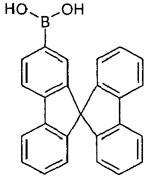 | 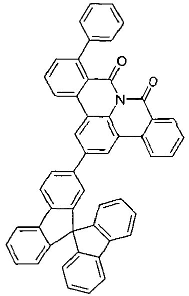 | 83% |
| | | 236389-21-2 | | |
| 8z | 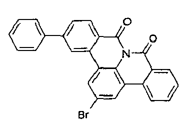 | 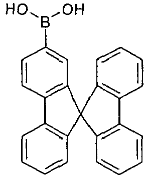 | 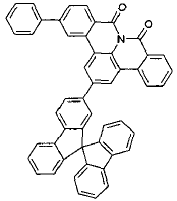 | 84% |
| | | 236389-21-2 | | |

Analog werden folgende Verbindungen mit 0.5 eq. Brom erhalten:

| **Bsp.** | **Edukt 1** | **Edukt 2** | **Produkt** | **Ausbeute** |
|---|---|---|---|---|
| 8w | 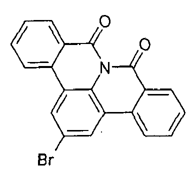 | 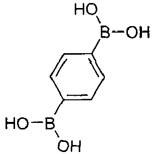 | 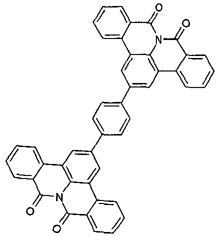 | 86% |
| | | [4612-26-4] | | |
| 8y | 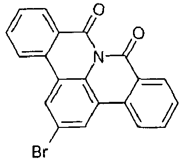 | 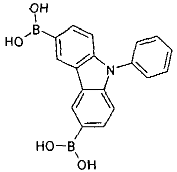 | 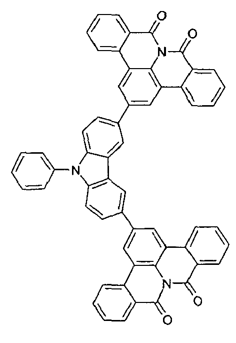 | 81% |
| | | [1135916-40-3] | | |

### Beispiel 9 : 2-(3-Phenyl-6-[(E)-((Z)-1-propenyl)-buta-1,3-dienyl]-carbazol-9-yl}-8a-aza-benzo[fg]naphthacen-8,9-dion

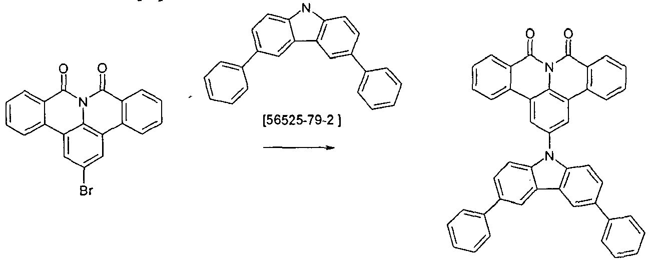

32 g (102-4 mmol) 3,6-Diphenyl-9H-carbazol, 42 g (112 mmol) 2-Brom-8a-aza-benzo[fg]naphthacen-8,9-dion und 2.3 (10-2 mmol)1,3-Di[2-pyridyl]-1,3-propandion, 28.3 g (204 mmol) Kaliumcarbonat und 1.9 g (10.2 mmol) Kupferiodid werden im 1000 ml DMF unter Rückfluss 90 h gerührt. Die Lösung wird mit Wasser verdünnt und mit Essigester zweimal extrahiert, die vereinigten organischen Phasen über Na₂SO₄ getrocknet und einrotiert und chromatographisch (EtOAc/Hexan: 2/3) gereinigt. Der Rückstand wird aus Toluol und aus Dichlormethan umkristallisiert und abschließend im Hochvakuum sublimiert, Reinheit beträgt 99.9 %. Die Ausbeute beträgt 46 g (75 mmol), entsprechend 68 % der Theorie.

Analog werden folgende Verbindungen erhalten:

| **Bsp.** | **Edukt 1** | **Edukt 2** | **Produkt** | **Ausbeute** |
|---|---|---|---|---|
| 9a | 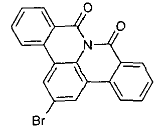 | 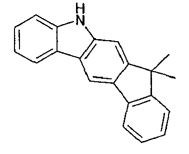 | 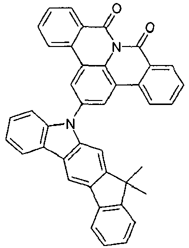 | 65% |
| | | [1257220-47-5] | | |

### Beispiel 10 : 2-(Bis-biphenyl-4-yl-amino)-8a-aza-benzo[fg]naphthacen-8,9-dion

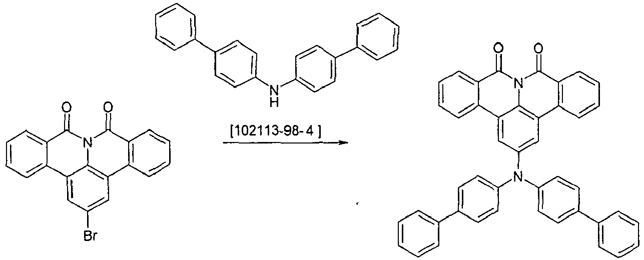

Unter Schutzgas werden 24.5 g (79.8 mmol) Bis-biphenyl-4-yl-amin, 32.7 g (87 mmol) 2-Brom-8a-aza-benzo[fg]naphthacen-8,9-dion, 15.9 ml (15.9 mmol) 1 mol/L Tri-tert-butylphosphin und 1.79 g (7.9 mmol) Palladiumacetat in 120 ml p-Xylol suspendiert. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99.9%, Ausbeute 44 g (72 mmol), 83 % der Theorie.

Analog werden folgende Verbindunaen erhalten:

| **Bsp.** | **Edukt 1** | **Edukt 2** | **Produkt** | **Ausbeute** |
|---|---|---|---|---|
| 10a | 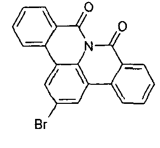 | 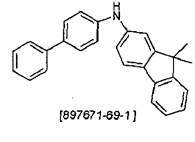 | 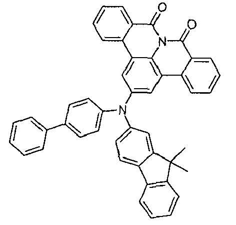 | 85% |
| 10b | 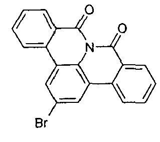 | 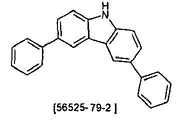 | 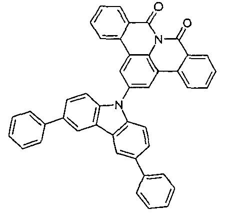 | 76% |

### Beispiel 11: -(2-Brom-benzyl)-3-phenyl-1,3-dihydro-benzoimidazol-2-on

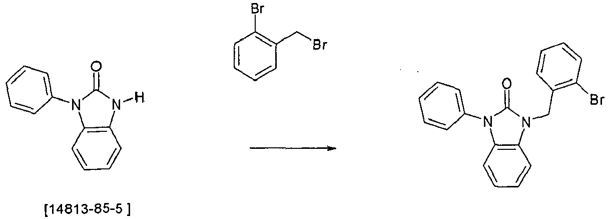

1-Phenyl-1,3-dihydro-benzoimidazol-2-on 52g (250 mmol) und 38 g (275 mmol) K₂CO₃ werden in 100 ml DMF vorgelegt. Nach 1 h bei Raumtemperatur wird eine Lösung von 62 g (250 mmol) 2-Brombenzylbromid in 500 mL DMF zugetropft. Das Reaktionsgemisch wird dann 25 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Die Ausbeute beträgt 65 g (70 %).

Analog werden folgende Verbindungen erhalten:

| **Bsp.** | **Edukt 1** | **Edukt 2** | **Produkt** | **Ausbeute** |
|---|---|---|---|---|
| 11a | 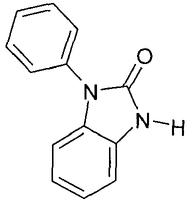 | 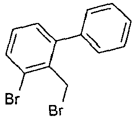 | 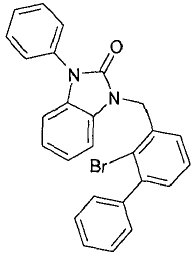 | 76% |
| | | 172976-02-2 | | |
| | [1225484-71-8] | | | |
| 11b | 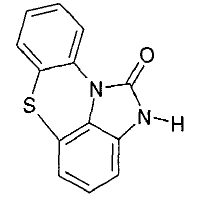 | 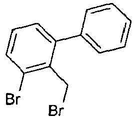 [172976-02-2] | 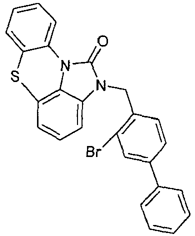 | 71% |
| | [30017-73-3] | | | |

Analog werden folgende Verbindungen mit 125 mmol 1,3-Dihydro-2H-benzimidazol-2-on [615-16-7] erhalten:

| **Bsp.** | **Edukt 1** | **Edukt 2** | **Produkt** | **Ausbeute.** |
|---|---|---|---|---|
| 11c | 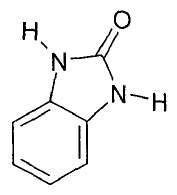 | 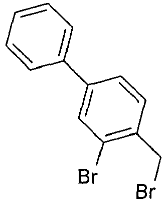 | 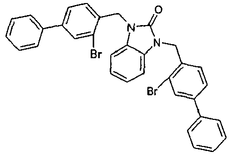 | 86% |
| | [615-16-7 ] | [1396865-04-5 ] | | |
| 11d | 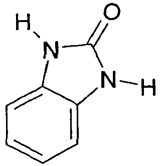 | 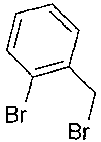 | 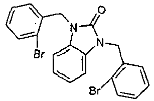 | 82% |
| | [615-16-7 ] | [3433-80-5 ] | | |

Die Cyclisierung erfolgt analog Beispiel 5:

| **Bsp.** | **Edukt 1** | **Produkt** | **Ausbeute** |
|---|---|---|---|
| 5d | 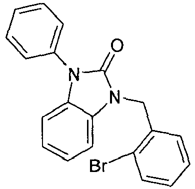 | 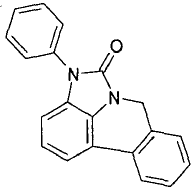 | 62% |
| 5e | 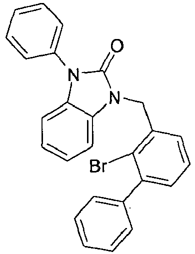 | 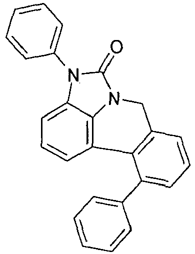 | 63% |
| 5f | 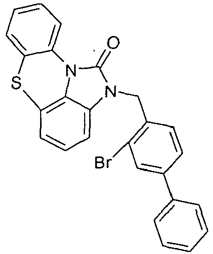 | 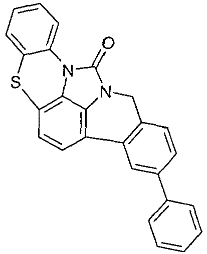 | 74% |
| 5h | 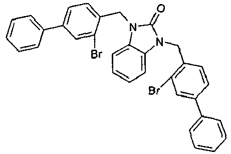 | 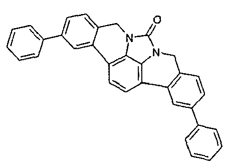 | 66% |
| 5j | 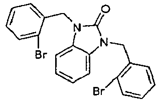 | 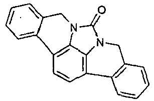 | 64% |

Die Weiteroxidation erfolgt analog Beispiel 6:

| **Bsp.** | **Edukt 1** | **Produkt** | **Ausbeute** |
|---|---|---|---|
| 6n | 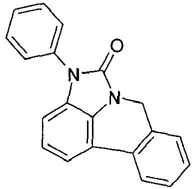 | 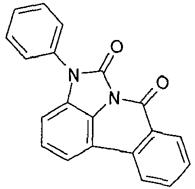 | 60% |
| 6o | 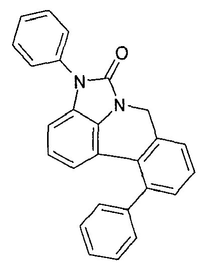 | 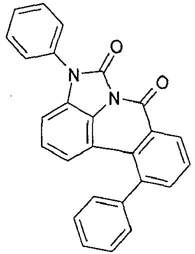 | 69% |
| 6p | 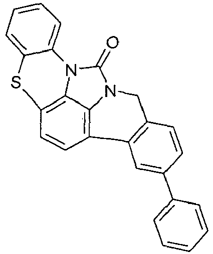 | 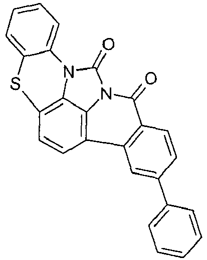 | 71% |
| 6q | 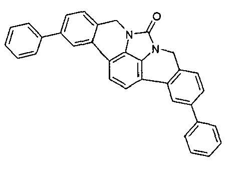 | 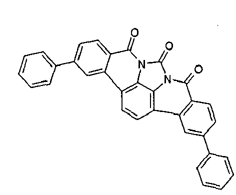 | 70% |
| 6r | 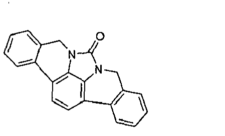 | 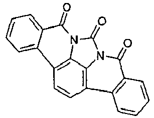 | 67% |

Die Bromierung erfolgt analog Beispiel 7:

| **Bsp.** | **Edukt 1** | **Produkt** | **Ausbeute** |
|---|---|---|---|
| 7n | 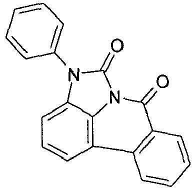 | 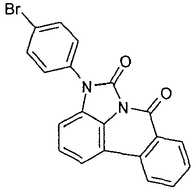 | 60% |
| 7o | 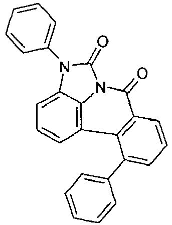 | 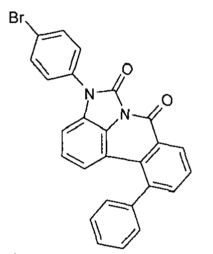 | 69% |
| 7p | 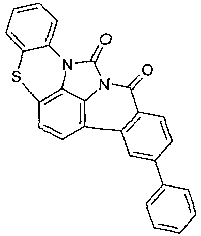 | 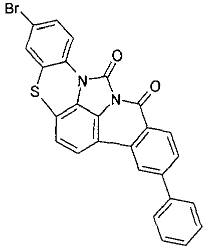 | 71% |
| 7q | 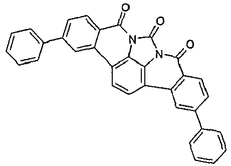 | 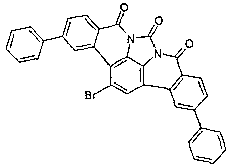 | 70% |
| 7r | 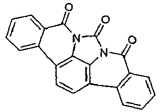 | 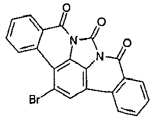 | 67% |

Über Ullmann-Reaktion analog zu Beispiel 9 werden folgende Verbindungen hergestellt:

| **Bsp.** | **Edukt 1** | **Edukt 2** | **Produkt** | **Ausbeute** |
|---|---|---|---|---|
| 9b | 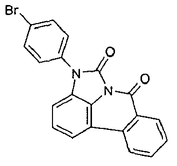 | 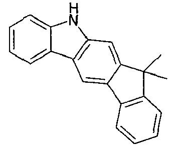 | 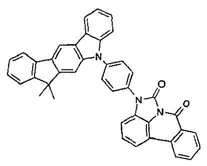 | 64% |
| | | [1257220-47-5] | | |
| 9c | 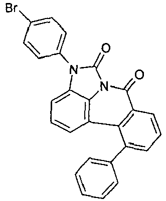 | 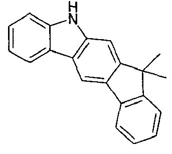 | 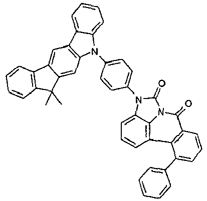 | 63% |
| | | [1257220-47-5] | | |
| 9d | 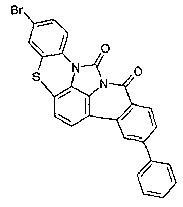 | 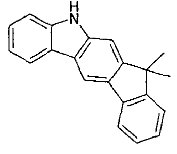 | 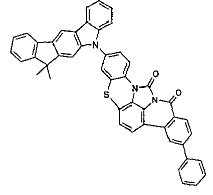 | 65% |
| | | [1257220-47-5] | | |
| 9e | 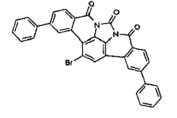 | 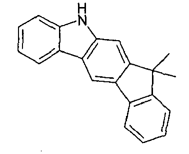 | 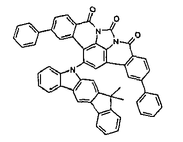 | 66% |
| | | [1257220-47-5] | | |
| 9f | 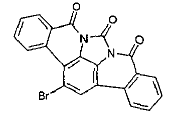 | 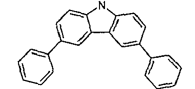 | 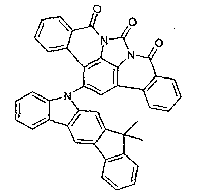 | 71% |
| | | [56525-79-2] | | |

### Beispiel 12: Herstellung der OLEDs

In den folgenden Beispielen E1 bis E27 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophen)poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt. Eine Bezeichnung wie "8a" bezieht sich hierbei auf die entsprechende Verbindung aus dem oben genannten Beispiel 8a. Dies gilt analog für alle verwendeten erfindungsgemäßen Materialien.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:8c:TER1 (55%:35%:10%) bedeutet hierbei, dass das Material IC1 in einem Volumenanteil von 55%, 8c in einem Anteil von 35% und TER1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 80% in Tabelle 2 bedeutet, dass die in Spalte LD angegebene Lebensdauer der Zeit entspricht, nach der die Anfangsleuchtdichte von 4000 cd/m² auf 3200 cd/m² absinkt. Analog bedeutet L0;j0 = 20 mA/cm², L1 = 70%, dass die Leuchtdichte bei Betrieb mit 20 mA/cm² nach der Zeit LD auf 70% ihres Anfangswertes absinkt.

Die Daten der verschiedenen OLEDs sind in Tabelle 2 zusammengefasst. Man erhält bei Einsatz der erfindungsgemäßen Verbindungen sowohl bei Verwendung als Elektronentransportmaterial (Beispiele E1, E2, E13, E17, E18) als auch Matrixmaterial für phopshoreszierende Emitter (restliche Beispiele) sehr gute Werte für Effizienz, Spannung und Lebensdauer. Dies gilt beim Einsatz als Einzelmatrix sowie in Mixed-Matrix Systemen in Kombination mit unterschiedlichen Materialien wie IC1, IC2, Cbz1. Insbesondere sind die hervorragenden Spannungen und damit Leistungseffizienzen bei gleichtzeitig sehr guter Lebensdauer hervorzuheben (siehe z. B. E10).

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| E1 | SpA1 140nm | HATCN 5nm | SpMA1 20nm | M1:D1 (95%:5%) 20nm | --- | 8k 30nm | LiQ 3nm |
| E2 | SpA1 140nm | HATCN 5nm | SpMA1 20nm | M1:D1 (95%:5%) 20nm | --- | 8s:LiQ (50%:50%) 30nm | --- |
| E3 | SpA1 70nm | HATCN 5nm | SpMA1 90nm | IC1:TEG1 (90%:10%) 30nm | --- | 8s:LiQ (50%:50%) 40nm | --- |
| E4 | SpA1 70nm | HATCN 5nm | SpMA1 90nm | 8:TEG1 (90%:10%) 30nm | --- | ST1:LiQ (50%:50%) 40nm | --- |
| E5 | SpA1 90nm | HATCN 5nm | SpMA1 130nm | 8a:TER1 (92%:8%) 40nm | --- | ST1:LiQ (50%:50%) 40nm | --- |
| E6 | SpA1 70nm | HATCN 5nm | SpMA1 90nm | 8b:TEG1 (90%:10%) 30nm | --- | ST1:LiQ (50%:50%) 40nm | --- |
| E7 | SpA1 90nm | HATCN 5nm | SpMA1 130nm | 8b:TER1 (92%:8%) 40nm | --- | ST1:LiQ (50%:50%) 40nm | --- |
| E8 | SpA1 90nm | HATCN 5nm | SpMA1 130nm | IC1:8c:TER1 (55%:35%:10%) 40nm | IC1 5nm | ST1:LiQ (50%:50%) 35nm | --- |
| E9 | SpA1 70nm | HATCN 5nm | SpMA1 90nm | 8e:TEG1 (90%:10%) 30nm | --- | ST1:LiQ (50%:50%) 40nm | --- |
| E10 | SpA1 70nm | HATCN 5nm | SpMA1 90nm | 8e:IC2:TEG1 (45%:45%:10%) 40nm | --- | ST1:LiQ (50%:50%) 30nm | --- |
| E11 | SpA1 70nm | HATCN 5nm | SpMA1 90nm | 8e:Cbz1:TEG1 (60%:35%:5%) 30nm | IC1 10nm | ST1:LiQ (50%:50%) 30nm | --- |
| E12 | SpA1 70nm | HATCN 5nm | SpMA1 90nm | 8k:TEG1 (90%:10%) 30nm | --- | ST1:LiQ (50%:50%) 40nm | --- |
| E13 | SpA1 70nm | HATCN 5nm | SpMA1 90nm | IC1:TEG1 (90%:10%) 30nm | --- | 8o 40nm | LiQ 3nm |
| E14 | SpA1 70nm | HATCN 5nm | SpMA1 90nm | 8r:TEG1 (90%:10%) 30nm | --- | ST1:LiQ (50%:50%) 40nm | --- |
| E15 | SpA1 70nm | HATCN 5nm | SpMA1 90nm | 8s:TEG1 (90%:10%) 30nm | --- | ST1:LiQ (50%:50%) 40nm | --- |
| E16 | SpA1 90nm | HATCN 5nm | SpMA1 130nm | 8s:TER1 (92%:8%) 40nm | --- | ST1:LiQ (50%:50%) 40nm | --- |
| E17 | SpA1 70nm | HATCN 5nm | SpMA1 90nm | IC1:TEG1 (90%:10%) 30nm | --- | 8s 40nm | LiF 1nm |
| E18 | SpA1 140nm | HATCN 5nm | SpMA1 20nm | M1:D1 (95%:5%) 20nm | --- | 8v 30nm | LiQ 3nm |
| E19 | SpA1 90nm | HATCN 5nm | SpMA1 130nm | 8w:TER1 (92%:8%) 40nm | --- | ST1:LiQ (50%:50%) 40nm | --- |
| E20 | SpA1 70nm | HATCN 5nm | SpMA1 90nm | 8w:TEG1 (90%:10%) 30nm | --- | ST1:LiQ (50%:50%) 40nm | --- |
| E21 | SpA1 90nm | HATCN 5nm | SpMA1 130nm | 8y:TER1 (92%:8%) 40nm | --- | ST1:LiQ (50%:50%) 40nm | --- |
| E22 | SpA1 90nm | HATCN 5nm | SpMA1 130nm | 9:TER1 (92%:8%) 40nm | --- | ST1:LiQ (50%:50%) 40nm | --- |
| E23 | SpA1 90nm | HATCN 5nm | SpMA1 130nm | 9a:TER1 (92%:8%) 40nm | --- | ST1:LiQ (50%:50%) 40nm | --- |
| E24 | SpA1 70nm | HATCN 5nm | SpMA1 90nm | 9b:TEG1 (90%:10%) 30nm | --- | ST1:LiQ (50%:50%) 40nm | --- |
| E25 | SpA1 70nm | HATCN 5nm | SpMA1 90nm | 9c:TEG1 (90%:10%) 30nm | --- | ST1:LiQ (50%:50%) 40nm | --- |
| E26 | SpA1 90nm | HATCN 5nm | SpMA1 130nm | 9f:TER1 (92%:8%) 40nm | --- | ST1:LiQ (50%:50%) 40nm | --- |
| E27 | SpA1 90nm | HATCN 5nm | SpMA1 130nm | IC2:10:TER1 (70%:20%:10%) 40nm | IC1 5nm | ST1:LiQ (50%:50%) 35nm | --- |

**Tabelle 2: Daten der OLEDs**

| Bsp. | U1000 (V) | SE1000 (cd/A) | LE1000 (lm/W) | EQE 1000 | CIE x/y bei 1000 cd/M 1 | L0;j0 | L1 % | LD (h) |
|---|---|---|---|---|---|---|---|---|
| E1 | 4.7 | 8.1 | 5.4 | 7.2% | 0.13/0.14 | 60mA/cm² | 70 | 200 |
| E2 | 4.5 | 8.5 | 5.9 | 7.3% | 0.13/0.14 | 60mA/cm² | 70 | 225 |
| E3 | 3.4 | 61 | 57 | 16.7% | 0.33/0.62 | 20mA/cm² | 70 | 220 |
| E4 | 3.3 | 56 | 53 | 15.3% | 0.32/0.62 | 20mA/cm² | 70 | 240 |
| E5 | 4.4 | 12.2 | 8.6 | 13.2% | 0.67/0.33 | 4000 cd/m² | 80 | 360 |
| E6 | 3.5 | 54 | 48 | 14.7% | 0.33/0.63 | 20mA/cm² | 70 | 195 |
| E7 | 4.6 | 11.0 | 7.5 | 11.9% | 0.67/0.33 | 4000 cd/m² | 80 | 345 |
| E8 | 4.1 | 12.6 | 9.6 | 13.6% | 0.67/0.33 | 4000 cd/m² | 80 | 490 |
| E9 | 3.0 | 54 | 57 | 14.8% | 0.32/0.62 | 20mA/cm² | 70 | 260 |
| E10 | 3.0 | 61 | 65 | 16.7% | 0.34/0.63 | 20mA/cm² | 80 | 310 |
| E11 | 3.3 | 63 | 61 | 17.4% | 0.32/0.62 | 20mA/cm² | 80 | 265 |
| E12 | 3.4 | 52 | 48 | 14.4% | 0.33/0.61 | 20mA/cm² | 80 | 180 |
| E13 | 3.6 | 57 | 50 | 15.6% | 0.32/0.62 | 10000 cd/m² | 70 | 220 |
| E14 | 3.2 | 55 | 54 | 15.0% | 0.32/0.62 | 20mA/cm² | 70 | 230 |
| E15 | 3.3 | 54 | 51 | 14.7% | 0.33/0.61 | 20mA/cm² | 80 | 145 |
| E16 | 4.6 | 10.6 | 7.3 | 11.4% | 0.67/0.33 | 4000 cd/m² | 80 | 310 |
| E17 | 3.4 | 59 | 55 | 16.1% | 0.33/0.62 | 20mA/cm² | 70 | 245 |
| E18 | 4.9 | 7.0 | 4.9 | 6.7% | 0.13/0.14 | 60mA/cm² | 70 | 230 |
| E19 | 3.1 | 56 | 56 | 15.5% | 0.34/0.61 | 20mA/cm² | 80 | 160 |
| E20 | 3.8 | 10.1 | 8.4 | 10.9% | 0.67/0.33 | 4000 cd/m² | 80 | 385 |
| E21 | 4.5 | 12 | 8.4 | 13.0% | 0.67/0.33 | 4000 cd/m² | 80 | 405 |
| E22 | 4.8 | 11 | 7.2 | 11.9% | 0.67/0.33 | 4000 cd/m² | 80 | 370 |
| E23 | 4.3 | 12.8 | 9.3 | 13.8% | 0.67/0.33 | 4000 cd/m² | 80 | 420 |
| E24 | 3.0 | 61 | 63 | 17.0% | 0.33/0.62 | 20mA/cm² | 80 | 165 |
| E25 | 3.2 | 59 | 58 | 16.4% | 0.32/0.62 | 20mA/cm² | 80 | 150 |
| E26 | 4.6 | 11.1 | 7.6 | 12.0% | 0.67/0.33 | 4000 cd/m² | 80 | 405 |
| E27 | 4.3 | 12.2 | 8.9 | 13.2% | 0.67/0.33 | 4000 cd/m² | 80 | 520 |

**Tabelle 3: Strukturformeln der Materialien für die OLEDs**

| | |
|---|---|
| 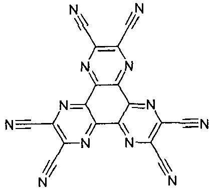 | 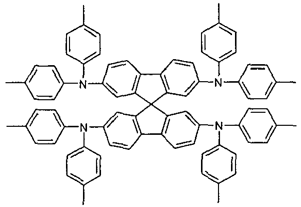 |
| HATCN | SpA1 |
| 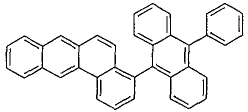 | 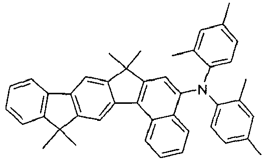 |
| M1 | D1 |
| 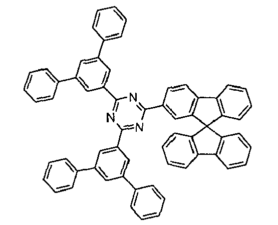 | 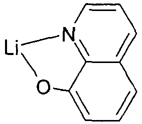 |
| ST1 | LiQ |
| 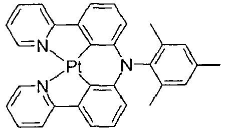 | 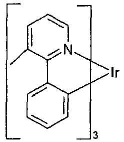 |
| TER1 | TEG1 |
| 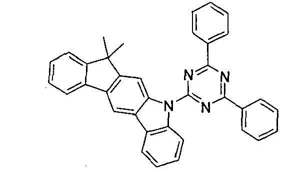 | 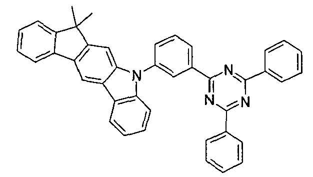 |
| IC1 | IC2 |
| 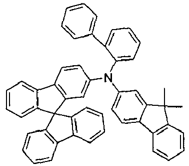 | 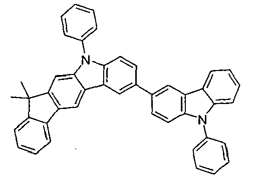 |
| SpMA1 | Cbz1 |

## Patentansprüche

1. Verbindung gemäß Formel (1), 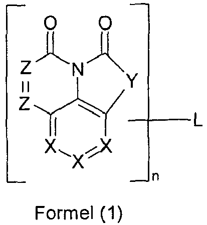 wobei für die verwendeten Symbole und Indizes gilt:
X ist bei jedem Auftreten gleich oder verschieden CR oder N; oder zwei benachbarte Gruppen X stehen zusammen für eine Gruppe, ausgewählt aus NR, O oder S, so dass ein Fünfring entsteht;
Y ist bei jedem Auftreten gleich oder verschieden Z=Z, NR, wobei R ungleich H ist, O oder S;
Z ist bei jedem Auftreten gleich oder verschieden CR oder N oder die benachbarten Gruppen Z=Z stehen zusammen für eine Gruppe der Formel (2), 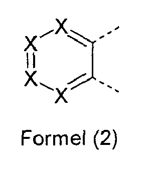 wobei X die oben genannten Bedeutungen aufweist und die gestrichelten Bindungen die Verknüpfung dieser Gruppe andeuten;
L ist für n = 1 nicht vorhanden und ist für n = 2 eine Einfachbindung oder eine bivalente Gruppe und für n = 3 eine trivalente Gruppe und für n = 4 eine tetravalente Gruppe und für n = 5 eine pentavalente Gruppe und für n = 6 eine hexavalente Gruppe; dabei ist L statt einer Gruppe R an einer beliebigen Stelle des Grundgerüsts gebunden;
R ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C=C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein;
R¹ ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine Alkylgruppe mit 1 bis 10 C-Atomen oder eine Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können;
n ist 1, 2, 3, 4, 5 oder 6;
wobei die folgende Verbindung von der Erfindung ausgenommen ist:

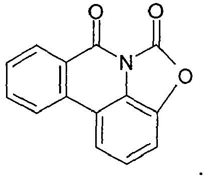

2. Verbindung nach Anspruch 1, ausgewählt aus den Verbindungen der Formel (5) und Formel (9), 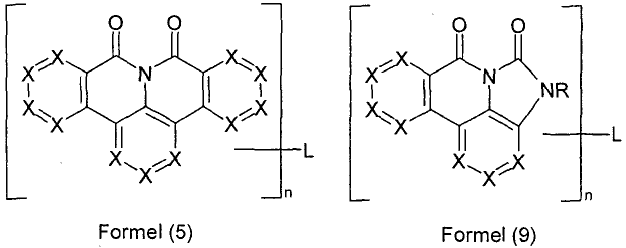 wobei die verwendeten Symbole und Indizes die in Anspruch 1 genannten Bedeutungen aufweisen und der Rest R am Stickstoff in Formel (9) ungleich H ist.

3. Verbindung nach Anspruch 1 oder 2, ausgewählt aus den Verbindungen der Formel (6), 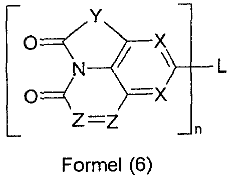 wobei n = 2 oder 3 ist und die weiteren Symbole die in Anspruch 1 genannten Bedeutungen aufweisen.

4. Verbindung nach einem oder mehreren der Ansprüche 1 bis 3, ausgewählt aus den Verbindungen der Formeln (7), (8), (10) und (11), 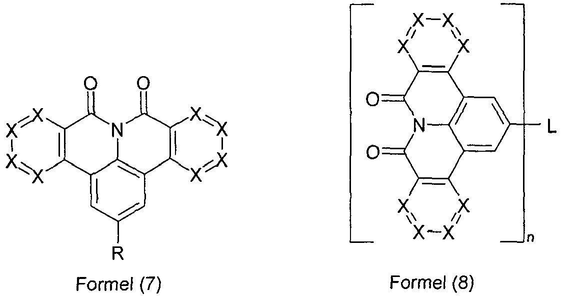 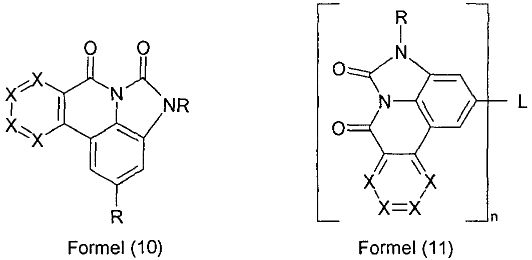 wobei n für 2 oder 3 steht, pro Cyclus maximal eine Gruppe X für N steht und die weiteren Gruppen X für CR stehen, die weiteren verwendeten Symbole die in Anspruch 1 genannten Bedeutungen aufweisen und der Rest R am Stickstoff in Formel (10) und (11) ungleich H ist.

5. Verbindung nach einem oder mehreren der Ansprüche 1 bis 4, ausgewählt aus den Verbindungen der Formeln (7a), (8a), (10a) und (11a), 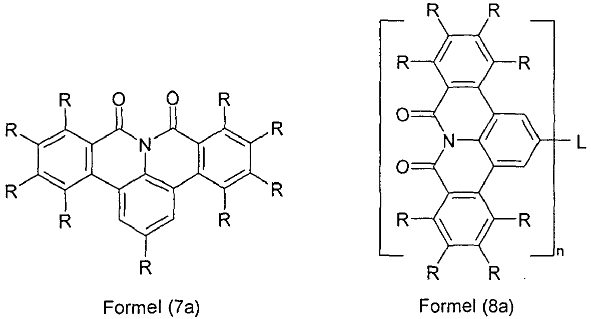 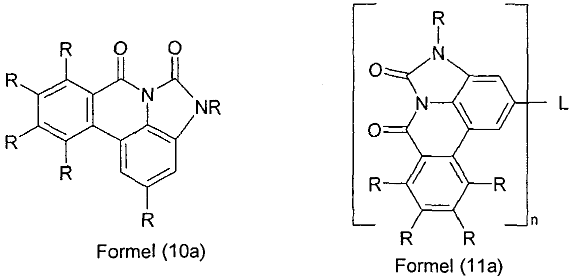 wobei n für 2 oder 3 steht, die weiteren verwendeten Symbole die in Anspruch 1 genannten Bedeutungen aufweisen und der Rest R am Stickstoff in Formel (10a) und (11a) ungleich H ist.

6. Verbindung nach einem oder mehreren der Ansprüche 1 bis 5, ausgewählt aus den Verbindungen der Formeln (7c), (8c), (10c) und (11 c), 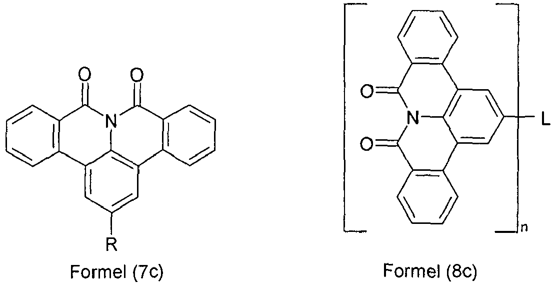 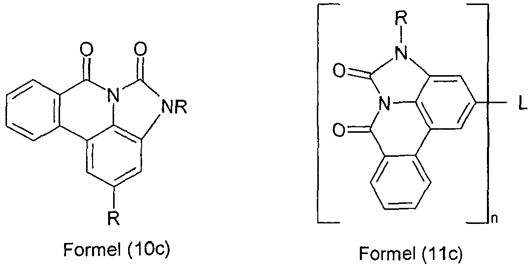 wobei n für 2 oder 3 steht, die weiteren verwendeten Symbole die in Anspruch 1 genannten Bedeutungen aufweisen und der Rest R am Stickstoff in Formel (10c) und (11c) ungleich H ist.

7. Verbindung nach einem oder mehreren der Ansprüche 1 bis 3, ausgewählt aus den Verbindungen der Formel (12), 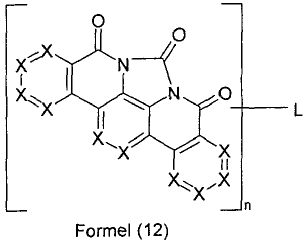 wobei die verwendeten Symbole und Indizes die in Anspruch 1 genannten Bedeutungen aufweisen.

8. Verbindung nach einem oder mehreren der Ansprüche 1 bis 7, **dadurch gekennzeichnet, dass** L für n = 2 ausgewählt ist aus einer Einfachbindung, CR₂, O, NR oder C(=O) oder für n = 3 für N steht oder für n ≥ 2 ausgewählt ist aus einem aromatischen oder heteroaromatischen Ringsystem mit 5 bis 24 aromatischen Ringatomen, das mit einem oder mehreren Resten R substituiert sein kann.

9. Verbindung nach einem oder mehreren der Ansprüche 1 bis 8, **dadurch gekennzeichnet, dass** mindestens ein Rest R ausgewählt ist aus der Gruppe bestehend aus einem aromatischen oder heteroaromatischen Ringsystem mit 5 bis 24 aromatischen Ringatomen, welches durch einen oder mehrere Reste R¹ substituiert sein kann, und/oder dass mindestens ein Rest R ausgewählt ist aus -C(=O)Ar¹ oder -P(=O)(Ar¹)₂ und/oder dass mindestens ein Rest R ausgewählt ist aus Triaryl- bzw. Heteroarylaminderivaten und/oder mindestens ein Substituent R steht für -N(Ar¹)₂

10. Verbindung nach einem oder mehreren der Ansprüche 1 bis 9, **dadurch gekennzeichnet, dass** mindestens eine Gruppe R ausgewählt ist aus der Gruppe bestehend aus Benzol, Biphenyl, Terphenyl, Quaterphenyl, Fluoren, Spirobifluoren, Naphthalin, Pyrrol, Furan, Thiophen, Indol, Benzofuran, Benzothiophen, Carbazol, Dibenzothiophen, Dibenzofuran, 1,3,5-Triazin, Pyridin, Pyrimidin, Pyrazin, Pyridazin, Indenocarbazol, verbrücktes Carbazol, Indolocarbazol, Anthracen, Phenanthren, Pyren, Triphenylen, Benzanthracen, Chinolin, Isochinolin, Phenanthridin, Phenanthrolin, Azacarbazol, Imidazol, Pyrazol, Thiazol, Oxazol, Oxadiazol, Triazol, Benzimidazol oder Kombinationen aus zwei, drei oder vier dieser Gruppen, wobei die Gruppen jeweils durch einen oder mehrere Reste R¹ substituiert sein können.

11. Formulierung, enthaltend mindestens eine Verbindung nach einem oder mehreren der Ansprüche 1 bis 10 und mindestens eine weitere Verbindung, insbesondere ein organisches Lösemittel.

12. Verwendung einer Verbindung nach einem oder mehreren der Ansprüche 1 bis 10 in einer elektronischen Vorrichtung.

13. Elektronische Vorrichtung, enthaltend mindestens eine Verbindung nach einem oder mehreren der Ansprüche 1 bis 10, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden und Organic Plasmon Emitting Devices.

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 10 als Matrixmaterial für einen fluoreszierenden oder phosphoreszierenden Emitter und/oder in einer Lochblockierschicht und/oder in einer Elektronentransportschicht eingesetzt wird.

## Claims

1. Compound of the formula (1), 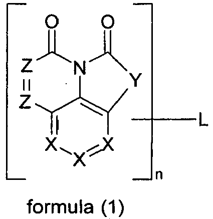 where the following applies to the symbols and indices used:
X is on each occurrence, identically or differently, CR or N; or two adjacent groups X together stand for a group selected from NR, O or S, resulting in the formation of a five-membered ring;
Y is on each occurrence, identically or differently, Z=Z, O, S or NR, where R is not equal to H;
Z is on each occurrence, identically or differently, CR or N or the adjacent groups Z=Z together stand for a group of the formula (2), 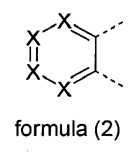 where X has the meanings given above and the dashed bonds indicate the linking of this group;
L is not present for n = 1 and is a single bond or a divalent group for n = 2 and a trivalent group for n = 3 and a tetravalent group for n = 4 and a pentavalent group for n = 5 and a hexavalent group for n = 6; L here is bonded at any desired point of the basic structure instead of a group R;
R is selected on each occurrence, identically or differently, from the group consisting of H, D, F, Cl, Br, I, CN, NO₂, N(Ar¹)₂, N(R¹)₂, C(=O)Ar¹, C(=O)R¹, P(=O)(Ar¹)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R¹, where one or more non-adjacent CH₂ groups may be replaced by R¹C=CR¹, C=C, Si(R¹)₂, C=O, C=S, C=NR¹, P(=O)(R¹), SO, SO₂, NR¹, O, S or CONR¹ and where one or more H atoms may be replaced by D, F, Cl, Br, I, CN or NO₂, an aromatic or heteroaromatic ring system having 5 to 60 aromatic ring atoms, which may in each case be substituted by one or more radicals R¹, an aryloxy or heteroaryloxy group having 5 to 60 aromatic ring atoms, which may be substituted by one or more radicals R¹, an aralkyl or heteroaralkyl group having 5 to 60 aromatic ring atoms, which may be substituted by one or more radicals R¹, or a combination of these systems, where two or more adjacent substituents R may optionally form a monocyclic or polycyclic, aliphatic, aromatic or heteroaromatic ring system, which may be substituted by one or more radicals R¹;
Ar¹ is on each occurrence, identically or differently, an aromatic or heteroaromatic ring system having 5-30 aromatic ring atoms, which may be substituted by one or more non-aromatic radicals R¹; two radicals Ar¹ here which are bonded to the same N atom or P atom may also be bridged to one another by a single bond or a bridge selected from N(R¹), C(R¹)₂ or O;
R¹ is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I, CN or an alkyl group having 1 to 10 C atoms or an alkenyl group having 2 to 10 C atoms, where two or more adjacent substituents R¹ may form a mono- or polycyclic, aliphatic, aromatic or hetero-aromatic ring system with one another;
n is 1, 2, 3, 4, 5 or 6;
where the following compound is excluded from the invention:

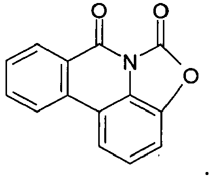

2. Compound according to Claim 1, selected from the compounds of the formula (5) and formula (9), 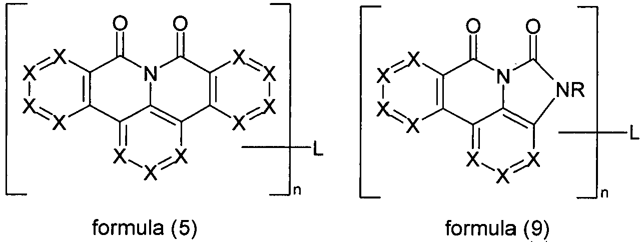 where the symbols and indices used have the meanings given in Claim 1 and the radical R on the nitrogen in formula (9) is not equal to H.

3. Compound according to Claim 1 or 2, selected from the compounds of the formula (6), 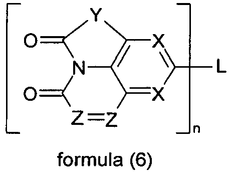 where n = 2 or 3 and the other symbols have the meanings given in Claim 1.

4. Compound according to one or more of Claims 1 to 3, selected from the compounds of the formulae (7), (8), (10) and (11), 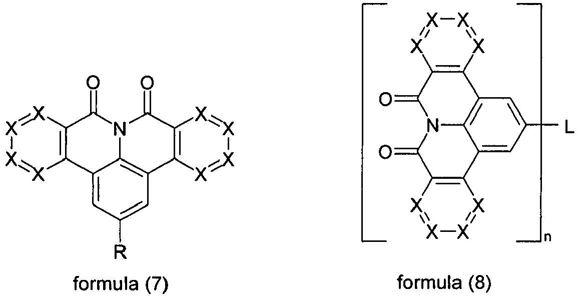 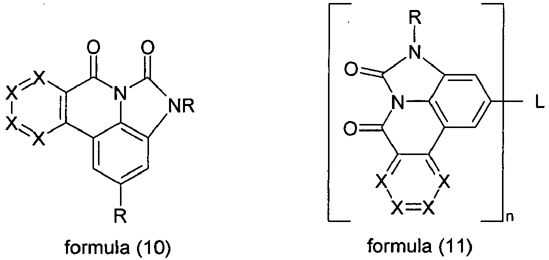 where n stands for 2 or 3, a maximum of one group X per ring stands for N and the other groups X stand for CR, the other symbols used have the meanings given in Claim 1 and the radical R on the nitrogen in formula (10) and (11) is not equal to H.

5. Compound according to one or more of Claims 1 to 4, selected from the compounds of the formulae (7a), (8a), (10a) and (11a), 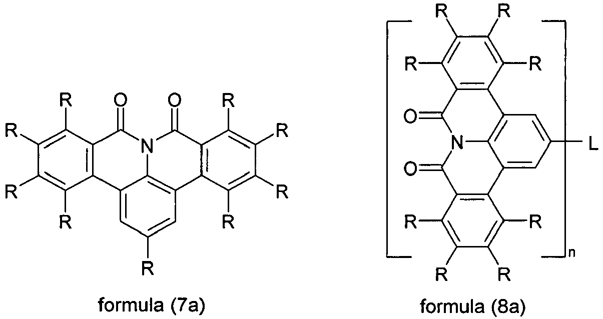 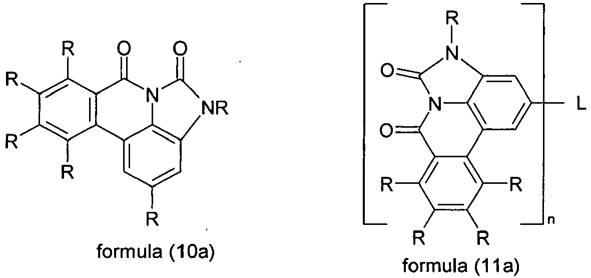 where n stands for 2 or 3, the other symbols used have the meanings given in Claim 1 and the radical R on the nitrogen in formula (10a) and (11a) is not equal to H.

6. Compound according to one or more of Claims 1 to 5, selected from the compounds of the formulae (7c), (8c), (10c) and (11c), 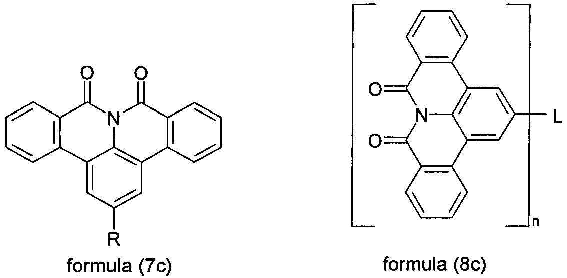 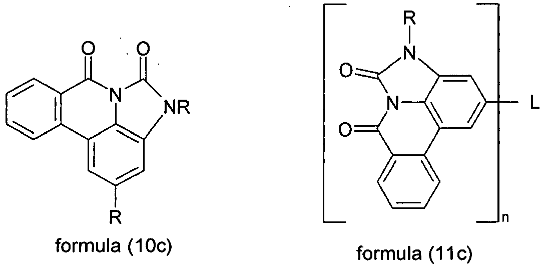 where n stands for 2 or 3, the other symbols used have the meanings given in Claim 1 and the radical R on the nitrogen in formula (10c) and (11c) is not equal to H.

7. Compound according to one or more of Claims 1 to 3, selected from the compounds of the formula (12), 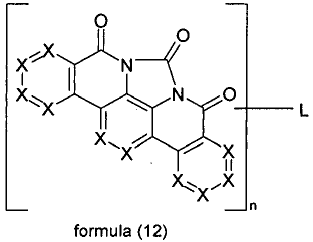 where the symbols and indices used have the meanings given in Claim 1.

8. Compound according to one or more of Claims 1 to 7, **characterised in that** L, for n = 2, is selected from a single bond, CR₂, O, NR or C(=O) or, for n = 3, stands for N or, for n ≥ 2, is selected from an aromatic or heteroaromatic ring system having 5 to 24 aromatic ring atoms, which may be substituted by one or more radicals R.

9. Compound according to one or more of Claims 1 to 8, **characterised in that** at least one radical R is selected from the group consisting of an aromatic or heteroaromatic ring system having 5 to 24 aromatic ring atoms, which may be substituted by one or more radicals R¹, and/or **in that** at least one radical R is selected from -C(=O)Ar¹ or -P(=O)(Ar¹)₂ and/or **in that** at least one radical R is selected from triaryl- or heteroarylamine derivatives and/or at least one substituent R stands for -N(Ar¹)₂

10. Compound according to one or more of Claims 1 to 9, **characterised in that** at least one group R is selected from the group consisting of benzene, biphenyl, terphenyl, quaterphenyl, fluorene, spirobifluorene, naphthalene, pyrrole, furan, thiophene, indole, benzofuran, benzothiophene, carbazole, dibenzothiophene, dibenzofuran, 1,3,5-triazine, pyridine, pyrimidine, pyrazine, pyridazine, indenocarbazole, bridged carbazole, indolocarbazole, anthracene, phenanthrene, pyrene, triphenylene, benzanthracene, quinoline, isoquinoline, phenanthridine, phenanthroline, azacarbazole, imidazole, pyrazole, thiazole, oxazole, oxadiazole, triazole, benzimidazole or combinations of two, three or four of these groups, where the groups may each be substituted by one or more radicals R¹.

11. Formulation comprising at least one compound according to one or more of Claims 1 to 10 and at least one further compound, in particular an organic solvent.

12. Use of a compound according to one or more of Claims 1 to 10 in an electronic device.

13. Electronic device comprising at least one compound according to one or more of Claims 1 to 10, in particular selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and organic plasmon emitting devices.

14. Electronic device according to Claim 13, which is an organic electroluminescent device, **characterised in that** the compound according to one or more of Claims 1 to 10 is employed as matrix material for a fluorescent or phosphorescent emitter and/or in a hole-blocking layer and/or in an electron-transport layer.

## Revendications

1. Composé de la formule (1) : 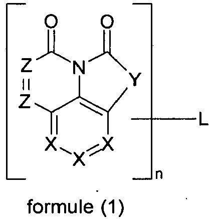 dans laquelle ce qui suit s'applique aux symboles et indices utilisés :
X est pour chaque occurrence, de manière identique ou différente, CR ou N; ou deux groupes X adjacents représentent en association un groupe choisi parmi NR, O ou S, d'où, en tant que résultat, la formation d'un cycle à cinq éléments ;
Y est pour chaque occurrence, de manière identique ou différente, Z=Z, O, S ou NR, où R n'est pas égal à H ;
Z est pour chaque occurrence, de manière identique ou différente, CR ou N ou les groupes Z=Z adjacents représentent en association un groupe de la formule (2) : 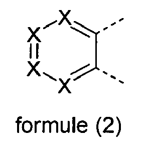 dans laquelle X présente les significations données ci avant et les liens en pointillés indiquent la liaison de ce groupe ;
L n'est pas présent pour n = 1 et est une liaison simple ou un groupe divalent pour n = 2 et un groupe trivalent pour n = 3 et un groupe tétravalent pour n = 4 et un groupe pentavalent pour n = 5 et un groupe hexavalent pour n = 6 ; L est ici lié en n'importe quel point souhaité de la structure de base en lieu et place d'un groupe R ;
R est choisi pour chaque occurrence, de manière identique ou différente, parmi le groupe constitué par H, D, F, CI, Br, I, CN, NO₂, N(Ar¹)₂, N(R¹)₂, C(=O)Ar¹, C(=O)R¹, P(=O)(Ar¹)₂, un groupe alkyle, alcoxy ou thioalkyle en chaîne droite comportant 1 à 40 atome(s) de C ou un groupe alkyle, alcoxy ou thioalkyle ramifié ou cyclique comportant 3 à 40 atomes de C ou un groupe alkényle ou alkynyle comportant 2 à 40 atomes de C, dont chacun peut être substitué par un radical ou plusieurs radicaux R¹, où un ou plusieurs groupe(s) CH₂ non adjacents peut/peuvent être remplacé(s) par R¹C=CR¹, C=C, Si(R¹)₂, C=O, C=S, C=NR¹, P(=O)(R¹), SO, SO₂, NR¹, O, S ou CONR¹ et où un ou plusieurs atome(s) de H peut/ peuvent être remplacé(s) par D, F, CI, Br, I, CN ou NO₂, un système de cycle aromatique ou hétéroaromatique comportant 5 à 60 atomes de cycle aromatique, lequel peut dans chaque cas être substitué par un radical ou plusieurs radicaux R¹, un groupe aryloxy ou hétéroaryloxy comportant 5 à 60 atomes de cycle aromatique, lequel peut être substitué par un radical ou plusieurs radicaux R¹, un groupe aralkyle ou hétéroaralkyle comportant 5 à 60 atomes de cycle aromatique, lequel peut être substitué par un radical ou plusieurs radicaux R¹, ou une combinaison de ces systèmes, où deux substituants R adjacents ou plus peuvent en option former un système de cycle aliphatique, aromatique ou hétéroaromatique monocyclique ou polycyclique, lequel peut être substitué par un radical ou plusieurs radicaux R¹ ;
Ar¹ est pour chaque occurrence, de manière identique ou différente, un système de cycle aromatique ou hétéroaromatique comportant 5-30 atomes de cycle aromatique, lequel peut être substitué par un ou plusieurs radical/radicaux R¹ non aromatique(s) ; deux radicaux Ar¹ ici qui sont liés au même atome de N ou au même atome de P peuvent également être pontés l'un à l'autre par une liaison simple ou un pont choisi parmi N(R¹), C(R¹)₂ ou O ;
R¹ est choisi parmi le groupe constitué par H, D, F, CN, un radical hydrocarbone aliphatique comportant 1 à 20 atome(s) de C, un système de cycle aromatique ou hétéroaromatique comportant 5 à 30 atomes de cycle aromatique, où un ou plusieurs atome(s) de H peut/peuvent être remplacé(s) par D, F, Cl, Br, I, CN ou un groupe alkyle comportant 1 à 10 atome(s) de C ou un groupe alkényle comportant 2 à 10 atomes de C, où deux substituants R¹ adjacents ou plus peuvent former un système de cycle aliphatique, aromatique ou hétéroaromatique monocyclique ou polycyclique l'un avec l'autre ou les uns avec les autres ;
n est 1, 2, 3, 4, 5 ou 6 ;
où le composé qui suit est exclus de l'invention:

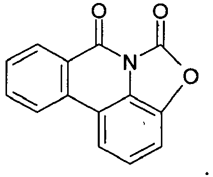

2. Composé selon la revendication 1, choisi parmi les composés de la formule (5) et de la formule (9) : 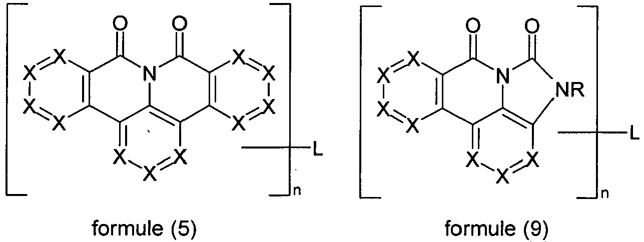 dans lesquelles les symboles et indices utilisés présentent les significations données selon la revendication 1 et le radical R sur l'azote dans la formule (9) n'est pas égal à H.

3. Composé selon la revendication 1 ou 2, choisi parmi les composés de la formule (6) : 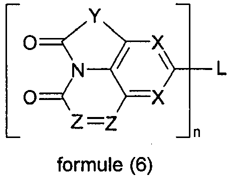 dans laquelle n = 2 ou 3 et les autres symboles présentent les significations données selon la revendication 1.

4. Composé selon une ou plusieurs des revendications 1 à 3, choisi parmi les composés des formules (7), (8), (10) et (11) : 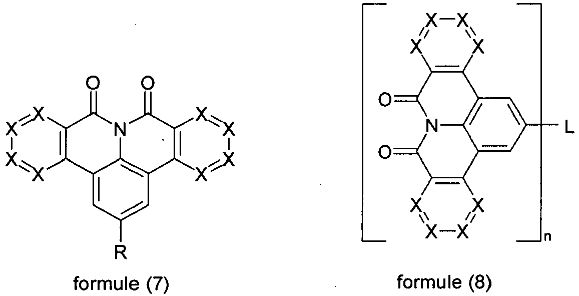 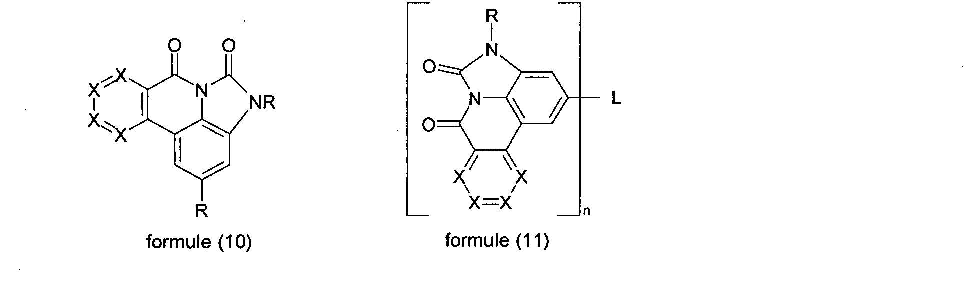 dans lesquelles n représente 2 or 3, un maximum d'un groupe X par cycle représente N et les autres groupes X représentent CR, les autres symboles utilisés présentent les significations données selon la revendication 1 et le radical R sur l'azote dans les formules (10) et (11) n'est pas égal à H.

5. Composé selon une ou plusieurs des revendications 1 à 4, choisi parmi les composés des formules (7a), (8a), (10a) et (11 a) : R R 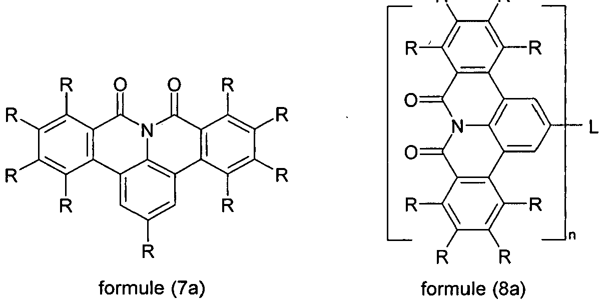 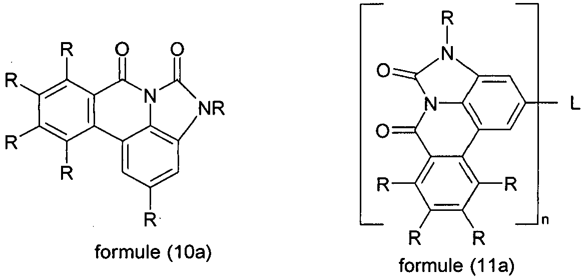 dans lesquelles n représente 2 ou 3, les autres symboles utilisés présentent les significations données selon la revendication 1 et le radical R sur l'azote dans les formules (10a) et (11 a) n'est pas égal à H.

6. Composé selon une ou plusieurs des revendications 1 à 5, choisi parmi les composés des formules (7c), (8c), (10c) et (11 c) : 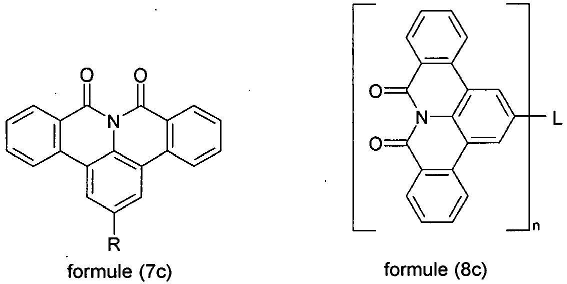 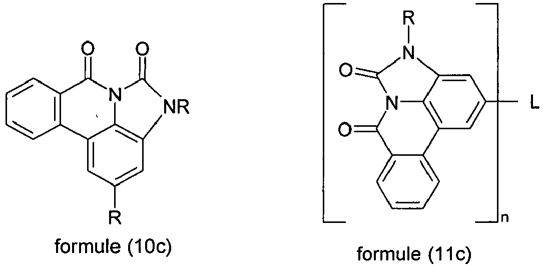 dans lesquelles n représente 2 ou 3, les autres symboles utilisés présentent les significations données selon la revendication 1 et le radical R sur l'azote dans les formules (10c) et (11c) n'est pas égal à H.

7. Composé selon une ou plusieurs des revendications 1 à 3, choisi parmi les composés de la formule (12) : 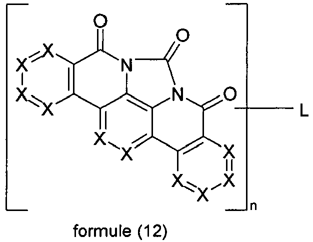 dans laquelle les symboles et indices utilisés présentent les significations données selon la revendication 1.

8. Composé selon une ou plusieurs des revendications 1 à 7, **caractérisé en ce que** L, pour n = 2, est choisi parmi une liaison simple, CR₂, O, NR ou C(=O) ou, pour n = 3, représente N ou, pour n ≥ 2, est choisi parmi un système de cycle aromatique ou hétéroaromatique comportant 5 à 24 atomes de cycle aromatique, lequel peut être substitué par un radical ou plusieurs radicaux R.

9. Composé selon une ou plusieurs des revendications 1 à 8, **caractérisé en ce qu'**au moins un radical R est choisi parmi le groupe constitué par un système de cycle aromatique ou hétéroaromatique comportant 5 à 24 atomes de cycle aromatique, lequel peut être substitué par un radical ou plusieurs radicaux R¹, et/ou **en ce qu'**au moins un radical R est choisi parmi -C(=O)Ar¹ ou -P(=O)(Ar¹)₂ et/ou **en ce qu'**au moins un radical R est choisi parmi des dérivés de triarylamine ou d'hétéroarylamine et/ou au moins un substituant R représente -N(Ar¹)₂.

10. Composé selon une ou plusieurs des revendications 1 à 9, **caractérisé en ce qu'**au moins un groupe R est choisi parmi le groupe constitué par benzène, biphényle, terphényle, quaterphényle, fluorène, spirobifluorène, naphtalène, pyrrole, furan, thiophène, indole, benzofuran, benzothiophène, carbazole, dibenzothiophène, dibenzofuran, 1,3,5-triazine, pyridine, pyrimidine, pyrazine, pyridazine, indénocarbazole, carbazole ponté, indolocarbazole, anthracène, phénanthrène, pyrène, triphénylène, benzanthracène, quinoline, isoquinoline, phénanthridine, phénanthroline, azacarbazole, imidazole, pyrazole, thiazole, oxazole, oxadiazole, triazole, benzimidazole ou des combinaisons de deux, trois ou quatre de ces groupes, où les groupes peuvent chacun être substitués par un radical ou plusieurs radicaux R¹.

11. Formulation comprenant au moins un composé selon une ou plusieurs des revendications 1 à 10 et au moins un autre composé, en particulier un solvant organique.

12. Utilisation d'un composé selon une ou plusieurs des revendications 1 à 10 dans un dispositif électronique.

13. Dispositif électronique comprenant au moins un composé selon une ou plusieurs des revendications 1 à 10, en particulier choisi parmi le groupe constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorants organiques, les détecteurs optiques organiques, les photorécepteurs organiques, les dispositifs à extinction de champ organiques, les cellules électrochimiques à émission de lumière organiques, les diodes laser organiques et les dispositifs à émission de plasmons organiques.

14. Dispositif électronique selon la revendication 13, lequel est un dispositif électroluminescent organique, **caractérisé en ce que** le composé selon une ou plusieurs des revendications 1 à 10 est utilisé en tant que matériau de matrice pour un émetteur fluorescent ou phosphorescent et/ou dans une couche de blocage de trous et/ou dans une couche de transport d'électrons.
